(19) Europäisches Patentamt
European Patent Office
Office européen des brevets

(11) Veröffentlichungsnummer: **0 663 397 A2**

(12) **EUROPÄISCHE PATENTANMELDUNG**

(21) Anmeldenummer: **94119744.4**

(22) Anmeldetag: **14.12.94**

(51) Int. Cl.[6]: **C07D 277/62**, A01N 25/32, C07D 263/56, C07D 235/08, C07D 237/28, C07D 239/74

(30) Priorität: **23.12.93 DE 4344074**

(43) Veröffentlichungstag der Anmeldung: **19.07.95 Patentblatt 95/29**

(84) Benannte Vertragsstaaten: **DE FR GB IT**

(71) Anmelder: **Hoechst Schering AgrEvo GmbH**
**Miraustrasse 54**
**D-13509 Berlin (DE)**

(72) Erfinder: **Voss, Olaf, Dr.**
**Im Hirtengrund 2**
**D-64297 Darmstadt (DE)**
Erfinder: **Willms, Lothar, Dr.**
**Königsteiner Strasse 59**
**D-65719 Hofheim (DE)**
Erfinder: **Bauer, Klaus, Dr.**
**Doorner Strasse 53d**
**D-63456 Hanau (DE)**
Erfinder: **Bieringer, Hermann, Dr.**
**Eichenweg 26**
**D-65817 Eppstein (DE)**

(54) **Bicyclische Heteroarylverbindungen, Verfahren zu deren Herstellung, diese enthaltende Mittel und deren Verwendung als Safener.**

(57) Verbindungen der Formel (I) und deren Salze,

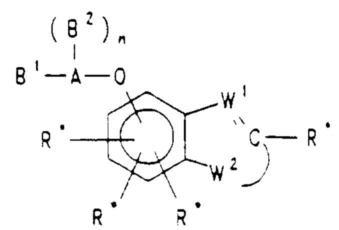

(I)

worin

| | |
|---|---|
| $W^1$ | N oder $CR^*$ bedeutet und $W^2$ O, S, NH, $NR^{**}$, N = N, N = $CR^*$ oder $CR^*$ = N bedeutet und die Summe der Heteroringatome aus den Gruppen $W^1$ und |
| $W^2$ | 2 oder 3 ist sowie |
| A | ein divalenter Kohlenwasserstoffrest mit 1 bis 12 C-Atomen, |
| $B^1$,$B^2$ | Carboxy oder dessen Derivate oder Formyl oder dessen Derivate oder ein Acylrest der Formel -CO-R, worin R ein aliphatischer, araliphatischer oder aromatischer Rest ist, der noch weitere funktionelle Gruppen enthalten und insgesamt bis zu 24 C-Atome aufweisen kann, |
| n | 0 oder 1, |
| $R^*$ | jeweils unabhängig von den anderen Resten $R^*$ H, Hal, $(C_1\text{-}C_8)$-Haloalkyl, $(C_1\text{-}C_8)$-Haloalkoxy, $(C_1\text{-}C_8)$-Alkyl, $(C_1\text{-}C_8)$-Alkoxy, $NO_2$, $NH_2$ oder Amino, das durch 1 oder 2 Reste aus der Gruppe $(C_1\text{-}C_4)$-alkyl und Acyl mit 1-10 C-Atomen substituiert ist, oder CN, $(C_1\text{-}C_8)$-Alkylthio oder $(C_1\text{-}C_8)$-Alkylsulfonyl oder (subst.) Phenyl, und |
| $R^{**}$ | $(C_1\text{-}C_8)$-Alkyl, $(C_1\text{-}C_8)$-Haloalkyl, $(C_1\text{-}C_8)$-Alkylthio, $(C_1\text{-}C_8)$-Alkoxy, $(C_1\text{-}C_8)$-Haloalkoxy, $(C_1\text{-}C_8)$-Alkyl- |

EP 0 663 397 A2

sulfonyl, Cyano-($C_1$-$C_8$)-alkyl,[($C_1$-$C_8$)-Alkyl]-carbonyl oder [($C_1$-$C_8$)-Alkoxy]-carbonyl
bedeuten und genauer in Anspruch 1 definiert sind, eignen sich erfindungsgemäß als Safener zur Reduzierung phytotoxischer Effekte von Pflanzenschutzmitteln (z. B. Herbiziden) an Kulturpflanzen. Die teils neuen teils bekannten Verbindungen sind analog bekannten Verfahren herstellbar.

Die Erfindung betrifft das technische Gebiet der Pflanzenschutzmittel, insbesondere Wirkstoff-Antidot-Kombinationen, die hervorragend für den Einsatz gegen konkurrierende Schadpflanzen in Nutzpflanzenkulturen geeignet sind.

Bei der Anwendung von Pflanzenbehandlungsmitteln, insbesondere bei der Anwendung von Herbiziden, können unerwünschte Schäden an den behandelten Kulturpflanzen auftreten. Viele Herbizide sind nicht voll verträglich (selektiv) mit einigen wichtigen Kulturpflanzen, wie Mais, Reis oder Getreide, so daß ihrem Einsatz enge Grenzen gesetzt sind. Sie können manchmal überhaupt nicht oder nur in solch geringen Aufwandmengen eingesetzt werden, daß die erwünschte breite herbizide Wirksamkeit gegen die Schadpflanzen nicht gewährleistet ist.

Aus EP-A-154 153 sind Aryloxy-Verbindungen als Safener für Phenoxyphenoxy- sowie Heteroaryloxyphenoxy-herbizide bekannt.

EP-A-293 062 beschreibt die Verwendung von Aryloxy-Verbindungen als Safener für Cyclohexandionherbizide.

Aus EP-A-94 349 ist die Verwendung von Chinolin-8-oxy-alkancarbonsäureestern als Safener für Herbizide aus verschiedenen Strukturklassen bekannt.

Aus DE-2637886 ist bereits die Verwendung von 3-Pyridyloxy-alkanamiden als Safener für Herbizide aus der Triazin-, Carbamat- und Haloacetanilid-Reihe bekannt.

Es wurde nun gefunden, daß sich überraschenderweise bicyclische Heteroarylderivate der nachstehenden Formel (I) hervorragend dazu eignen, Kulturpflanzen gegen schädigende Wirkungen von aggressiven Agrarchemikalien, insbesondere Herbiziden, zu schützen.

Gegenstand der Erfindung ist somit die Verwendung der Verbindungen der Formel (I) oder deren Salze,

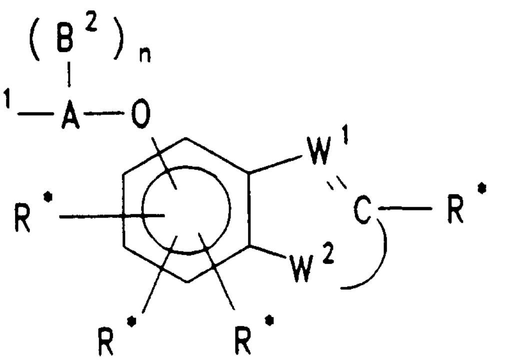

(I)

worin

| | |
|---|---|
| $W^1$ | N oder $CR^*$ und |
| $W^2$ | O, S, NH, $NR^{**}$, N = N, N = $CR^*$ oder $CR^*$ = N bedeuten und die Summe der Heteroringatome aus den Gruppen $W^1$ und $W^2$ 2 oder 3 ist, sowie |
| A | ein divalenter Kohlenwasserstoffrest mit 1 bis 12, vorzugsweise 1 bis 8 C-Atomen, beispielsweise ($C_1$-$C_6$)-Alkandiyl oder ($C_2$-$C_8$)-Alkendiyl, |
| $B^1$, $B^2$ | jeweils unabhängig voneinander Carboxy, Derivate der Carboxygruppe aus der Gruppe Ester, Thioester, Nitrile, Imide, Anhydride, Halogenide, Amide, Hydrazide, Imidsäure, Hydrazonsäure, Hydroxamsäure, Hydroximsäure, Amidin, Amidoxid und Derivate der letztgenannten 9 funktionellen Reste, die am N-Atom bzw. der Hydroxygruppe, weiter substituiert sind, oder Formyl, Derivate der Formylgruppe aus der Gruppe Acetale, Thioacetale, Imine, Hydrazon, Oxim und Derivate der letztgenannten 3 funktionellen Reste, die am N-Atom bzw. an der Hydroxygruppe weiter substituiert sind, oder ein Acylrest der Formel -CO-R, worin |
| R | ein aliphatischer, araliphatischer oder aromatischer Rest ist, der noch weitere funktionelle Gruppen enthalten und insgesamt bis zu 24 C-Atome aufweisen kann, |
| n | 0 oder 1, |
| $R^*$ | jeweils unabhängig von den anderen Resten $R^*$ Wasserstoff, Halogen, Halogen-($C_1$-$C_8$)-alkyl, Halogen-($C_1$-$C_8$)-alkoxy, ($C_1$-$C_8$)-Alkyl, ($C_1$-$C_8$)-Alkoxy, Nitro, Amino oder Amino, das durch ein oder zwei Reste aus der Gruppe ($C_1$-$C_4$)-alkyl und Acyl mit 1 bis 10 C-Atomen substituiert ist, oder Cyano, ($C_1$-$C_8$)-Alkylthio, ($C_1$-$C_8$)-Alkylsulfonyl oder gegebenenfalls substituiertes Phenyl, |

vorzugsweise Wasserstoff, Halogen, $(C_1-C_6)$-Halogenalkyl, wie Trifluormethyl, $(C_1-C_6)$-Halogenalkoxy, wie Difluormethoxy, $(C_1-C_6)$-Alkyl, $(C_1-C_6)$-Alkoxy, $(C_1-C_6)$-Alkylthio, $(C_1-C_6)$-Alkylsulfonyl, Nitro, Amino, Mono-$(C_1-C_4)$-alkylamino, Di-$(C_1-C_4)$-alkylamino, $(C_1-C_4)$-Alkanoylamino, N-$(C_1-C_4)$-Alkanoyl-N-$(C_1-C_4)$-alkyl-amino, Benzoylamino, Benzyloxycarbonylamino, Cyano oder gegebenenfalls substituiertes Phenyl,

und

$R'''$ $(C_1-C_8)$-Alkyl, Halogen-$(C_1-C_8)$-alkyl, $(C_1-C_8)$-Alkylthio, $(C_1-C_8)$-Alkoxy, Halogen-$(C_1-C_8)$-alkoxy, $(C_1-C_8)$-Alkylsulfonyl, Cyano-$(C_1-C_8)$-alkyl, $[(C_1-C_8)$-Alkyl]-carbonyl oder $[(C_1-C_8)$-Alkoxy]-carbonyl

bedeuten,

als Safener zur Vermeidung oder Reduzierung phytotoxischer Effekte von Pflanzenschutzmitteln an Kulturpflanzen.

Geeignete Safener sind beispielsweise erfindungsgemäße Verbindungen der Formel (I), worin

A $(C_1-C_6)$-Alkandiyl oder $(C_3-C_8)$-Alkendiyl,

$B^1$, $B^2$ jeweils unabhängig voneinander einen Rest der Formel

$-COOR^1$,

$-COSR^1$,

$-CO-ON = CR^2R^3$,

$-CO-NR^4N = CR^2R^3$,

$-CONR^1R^4$,

$-CO-R^1$ oder

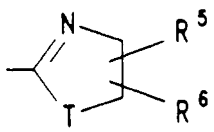 , 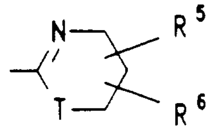 , 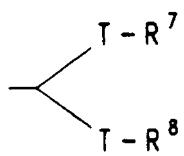 oder 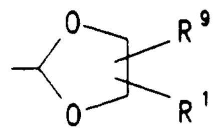 ,

$R^1$ Wasserstoff, $(C_1-C_{18})$-Alkyl, $(C_3-C_{12})$-Cycloalkyl, $(C_3-C_{12})$-Heterocyclyl, $(C_2-C_{18})$-Alkenyl, $(C_2-C_8)$-Alkinyl, Aryl oder Heteroaryl,

wobei jeder der vorstehenden C-haltigen Reste unsubstituiert oder durch einen oder mehrere, gleiche oder verschiedene Substituenten aus der Gruppe Halogen, Halogen-$(C_1-C_8)$-alkoxy, Nitro, Cyano, Hydroxy, $(C_1-C_8)$-Alkoxy und $(C_1-C_8)$-Alkoxy, worin eine oder mehrere nicht nebeneinanderliegende $CH_2$-Gruppen durch Sauerstoff ersetzt sind, und $(C_1-C_8)$-Alkyl und Halo-$(C_1-C_8)$-alkyl, letztere beiden nur bei cyclischen Resten, $(C_1-C_8)$-Alkylthio, $(C_1-C_6)$-Alkylsulfinyl, $(C_1-C_6)$-Alkylsulfonyl, $(C_2-C_8)$-Alkenylthio, $(C_2-C_8)$-Alkinylthio, $(C_2-C_8)$-Alkenyloxy, $(C_2-C_8)$-Alkinyloxy, $(C_3-C_7)$-Cycloalkyl, $(C_3-C_7)$-Cycloalkoxy, $(C_1-C_8)$-Alkoxycarbonyl, $(C_2-C_8)$-Alkenyloxycarbonyl, $(C_2-C_8)$-Alkinyloxycarbonyl, $(C_1-C_8)$-Alkylthiocarbonyl, $(C_1-C_8)$-Alkylcarbonyl, Formyl, $(C_2-C_8)$-Alkenylcarbonyl, $(C_2-C_8)$-Alkinylcarbonyl, Hydroxyimino, $(C_1-C_4)$-Alkylimino, $(C_1-C_4)$-Alkoxyimino, $(C_1-C_8)$-Alkylcarbonylamino, $(C_2-C_8)$-Alkenylcarbonylamino, $(C_2-C_8)$-Alkinylcarbonylamino, Carbamoyl, $(C_1-C_8)$-Alkylcarbamoyl, Di-$(C_1-C_6)$-Alkylcarbamoyl, $(C_2-C_6)$-Alkenylcarbamoyl, $(C_2-C_6)$-Alkinylcarbamoyl, $(C_1-C_8)$-Alkoxycarbonylamino, $(C_1-C_8)$-Alkyl-amino-carbonylamino, Di-$(C_1-C_8)$-Alkylaminocarbonylamino, $(C_1-C_8)$-Alkoxycarbonyloxy, $(C_1-C_8)$-Alkylcarbonyloxy, das unsubstituiert oder durch Halogen, Nitro, $(C_1-C_4)$-Alkoxy oder gegebenenfalls substituiertes Phenyl substituiert ist, $(C_2-C_6)$-Alkenylcarbonyloxy, $(C_2-C_6)$-Alkinylcarbonyloxy, Phenyl, Phenyl-$(C_1-C_6)$-alkoxy, Phenyl-$(C_1-C_6)$-alkoxycarbonyl, Phenoxy, Phenoxy-$(C_1-C_6)$-alkoxy, Phenoxycarbonyl, Phenoxy-$(C_1-C_6)$-alkoxycarbonyl, Phenylcarbonyloxy, Phenylcarbonylamino, Phenyl-$(C_1-C_6)$-alkylcarbonylamino, wobei die letztgenannten 10 Reste im Phenylring unsubstituiert oder ein- oder mehrfach, vorzugsweise bis zu dreifach, durch gleiche oder verschiedene Reste aus der Gruppe Halogen, $(C_1-C_4)$-Alkyl, $(C_1-C_4)$-Alkoxy, $(C_1-C_4)$-Halogenalkyl, $(C_1-C_4)$-Halogenalkoxy und Nitro substituiert sind, und Reste der Formeln, $-SiR^2R^3R^4$, $-O-SiR^2R^3R^4$, $R^2R^3R^4Si-(C_1-C_6)$-alkoxy, $-CO-O-NR^2R^3$, $-O-N = CR^2R^3$, $-N = CR^2R^3$, $O-(CH_2)_m-CH(OR^2)OR^3$, $R'O-CHR''-CH(OR')-(C_1-C_6)$-alkoxy, $-NR^2R^3$, $-CO-NR^2R^3$, $-O-CO-NR^2R^3$ und einen drei- bis siebengliedrigen, gegebenenfalls benzokondensierten und gegebenenfalls substituierten, vorzugsweise bis zu dreifach

durch Halogen und/oder $(C_1-C_4)$-Alkyl substituierten, gesättigten oder ungesättigten heterocyclischen Rest mit bis zu drei gleichen oder verschiedenen Heteroatomen aus der Reihe S, O und N
substituiert ist,

| | |
|---|---|
| R' | unabhängig voneinander $(C_1-C_4)$-Alkyl oder paarweise zusammen einen $(C_1-C_6)$-Alkandiylrest und |
| R'' | Wasserstoff oder $(C_1-C_4)$-Alkyl, |
| $R^2$ und $R^3$ | jeweils unabhängig voneinander Wasserstoff, gegebenenfalls substituiertes $(C_1-C_6)$-Alkyl, $(C_2-C_6)$-Alkenyl, $(C_2-C_6)$-Alkinyl, Phenyl-$(C_1-C_4)$-alkyl oder Phenyl, wobei die letztgenannten beiden Reste im Phenylrest unsubstituiert oder substituiert sind, oder |
| $R^2$ und $R^3$ | gemeinsam eine gegebenenfalls substituierte $(C_2-C_6)$-Alkandiylkette, |
| $R^4$ | Wasserstoff oder gegebenenfalls substituiertes $(C_1-C_4)$-Alkyl oder |
| $R^1$ und $R^4$ | gemeinsam eine Alkandiylkette mit 2 bis 5 C-Atomen, in der eine $CH_2$-Gruppe gegebenenfalls durch O, NH oder N-$(C_1-C_4)$-Alkyl ersetzt sein kann, |
| $R^5$ und $R^6$ | gleich oder verschieden sind und unabhängig voneinander Wasserstoff oder $(C_1-C_6)$-Alkyl, |
| $R^7$ und $R^8$ | gleich oder verschieden sind und unabhängig voneinander Wasserstoff oder $(C_1-C_6)$-Alkyl, das durch Halogen, $(C_1-C_4)$-Alkoxy oder Phenyl substituiert sein kann, |
| $R^9$ und $R^{10}$ | gleich oder verschieden sind und unabhängig voneinander Wasserstoff oder $(C_1-C_6)$-Alkyl, das durch Halogen, $(C_1-C_4)$-Alkoxy oder OH substituiert sein kann, |
| T | unabhängig voneinander Sauerstoff oder Schwefel und |
| m | eine ganze Zahl von 0 bis 6 bedeuten. |

In den obengenannten Verbindungen der Formel (I) und im folgenden sind, sofern im einzelnen nicht anders festgelegt, Alkyl, Alkenyl und Alkinyl geradkettig oder verzweigt; entsprechendes gilt für die substituierten Alkyl-, Alkenyl- und Alkinylreste wie Haloalkyl, Hydroxyalkyl, Alkoxycarbonyl etc. $(C_1-C_4)$-Alkyl bedeutet Alkyl mit 1 bis 4 C-Atomen. In den zusammengesetzten Begriffen wie $(C_1-C_4)$-Alkylcarbonyl bezieht sich die Zahl der C-Atome nur auf den Alkylrest und nicht auf die Summe aus den C-Atomen des Alkylrestes und der daran gebundenen Carbonylgruppe; entsprechendes gilt für andere zusammengesetzte Begriffe. Alkyl bedeutet z. B. Methyl, Ethyl, n- und i-Propyl, n-, i-, t- und 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl bedeutet z. B. Allyl, 1-Methylprop-2-en-1-yl, 2-Methyl-prop-2-en-1-yl, But-2-en-1-yl, But-3-en-1-yl, 1-Methyl-but-3-enyl und 1-Methyl-but-2-enyl; Alkinyl bedeutet z. B. Propargyl, But-2-in-1-yl, But-3-in-1-yl, 1-Methyl-but-3-inyl.

Unter substituiertem Alkyl oder Alkandiyl versteht man ein- oder mehrfach, vorzugsweise bis zu dreifach, insbesondere einfach, durch gleiche oder verschiedene Reste aus der Reihe Halogen, Hydroxy und $(C_1-C_6)$-Alkoxy substituiertes Alkyl bzw. Alkandiyl.

Halogen bedeutet Fluor, Chlor, Brom oder Iod, vorzugsweise Fluor, Chlor oder Brom, insbesondere Fluor oder Chlor. Haloalkyl, -alkenyl und -alkinyl bedeuten durch Halogen teilweise oder vollständig substituiertes Alkyl, Alkenyl bzw. Alkinyl, z.B. $CF_3$, $CHF_2$, $CH_2F$, $CF_3CF_2$, $CH_2FCHCl$, $CCl_3$, $CHCl_2$, $CH_2CH_2Cl$; Haloalkoxy ist z.B. $OCF_3$, $OCHF_2$, $OCH_2F$, $CF_3CF_2O$, $OCH_2CF_3$. Entsprechendes gilt für Haloalkenyl und andere durch Halogen substituierte Reste.

Aryl bedeutet beispielsweise Phenyl, Naphthyl, Tetrahydronaphthyl, Indenyl, Indanyl, Pentalenyl, Fluorenyl und ähnliches, vorzugsweise Phenyl; Aryloxy bedeutet vorzugsweise die den genannten Arylresten entsprechenden Oxy-Reste, insbesondere Phenoxy.

Ein drei- bis siebengliedriger wie oben erwähnter heterocyclischer Rest (oder Heterocyclyl) ist vorzugsweise von Benzol abgeleitet, wovon mindestens ein CH durch N oder mindestens zwei benachbarte CH durch ein NH, S oder O ersetzt sind. Der Rest kann benzokondensiert sein. Er ist gegebenenfalls teilweise oder vollständig hydriert. Es kommen insbesondere Reste wie Oxiranyl, Pyrrolidyl, Piperidyl, Dioxolanyl, Pyrazolyl, Morpholyl, Furyl, Tetrahydrofuryl, Indolyl, Azepinyl, Triazolyl, Thienyl und Oxazolyl in Frage.

Heteroaryl bzw. Heteroaryl in Heteroaryloxy bedeutet beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl, aber auch bicyclische oder polycyclische aromatische oder araliphatische Reste, z. B. Chinolinyl, Benzoxazolyl etc..

Substituiertes Aryl bzw. Aryloxy, Heteroaryl, Heteroaryloxy, Phenyl, Phenoxy, Benzyl, Benzyloxy bzw. substituierte bicyclische Reste mit aromatischen Anteilen bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkyl, Haloalkyl, Alkoxy, Haloalkoxy, Hydroxy, Amino, Nitro, Cyano, Alkoxycarbonyl, Alkylcarbonyl, Formyl, Carbamoyl, Mono- und Dialkylaminocarbonyl, Mono- und Dialkylamino, Alkylsulfinyl und Alkylsulfonyl bedeuten und bei Resten mit C-Atomen

solche mit 1 bis 4 C-Atomen, insbesondere 1 oder 2, bevorzugt sind. Bevorzugt sind dabei in der Regel Substituenten aus der Gruppe Halogen, z. B. Fluor und Chlor, $C_1$-$C_4$-Alkyl, vorzugsweise Methyl oder Ethyl, $C_1$-$C_4$-Haloalkyl, vorzugsweise Trifluormethyl, $C_1$-$C_4$-Alkoxy, vorzugsweise Methoxy oder Ethoxy, $C_1$-$C_4$-Haloalkoxy, Nitro und Cyano. Besonders bevorzugt sind dabei die Substituenten Methyl, Methoxy und Chlor.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, ($C_1$-$C_4$)-Alkyl, ($C_1$-$C_4$)-Alkoxy, ($C_1$-$C_4$)-Halogenalkyl, ($C_1$-$C_4$)-Halogenalkoxy und Nitro substituiert ist, z. B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Ein aliphatischer, araliphatischer oder aromatischer Rest ist vorzugsweise ein gesättigter oder ein- oder mehrfach ungesättigter Kohlenwasserstoffrest, Aryl, Arylalkyl, Heteroaryl oder Heteroarylalkyl, wobei der Rest gegebenenfalls noch substituiert ist.

Acyl bedeutet beispielsweise Formyl, Alkylcarbonyl wie ($C_1$-$C_4$-Alkyl)-carbonyl, Phenylcarbonyl, wobei der Phenylring substituiert sein kann, z. B. wie oben für Phenyl gezeigt, oder Alkyloxycarbonyl, Phenyloxycarbonyl, Benzyloxycarbonyl, Alkylsulfonyl und andere Acylreste von organischen Säuren.

Manche Verbindungen der Formel (I) enthalten ein oder mehrere asymmetrische C-Atome oder Doppelbindungen, die in der allgemeinen Formel (I) nicht gesondert angegeben sind. Die durch ihre spezifische Raumform definierten möglichen Stereoisomeren, wie Enantiomere, Diastereomere, E- und Z-Isomere sowie deren Gemische sind jedoch alle von der Formel (I) umfaßt.

Die Verbindungen der Formel (I) können Salze bilden, bei denen der Rest $R^1$ durch ein Äquivalent eines für die Landwirtschaft geeigneten Kations ersetzt wird. Diese Salze sind beispielsweise Metall-, insbesondere Alkali-oder Erdalkalisalze, aber auch Ammoniumsalze oder Salze mit organischen Aminen sowie Salze, die als Kationen Sulfonium- oder Phosphoniumionen enthalten.

Als Salzbildner eignen sich besonders Metalle und organische Stickstoffbasen, vor allem quartäre Ammoniumbasen. Hierbei kommen als zur Salzbildung geeignete Metalle Erdalkalimetalle, wie Magnesium oder Calcium, vor allem aber Alkalimetalle in Betracht, wie Lithium und insbesondere Kalium und Natrium.

Beispiele für zur Salzbildung geeignete Stickstoffbasen sind primäre, sekundäre oder tertiäre, aliphatische und aromatische, gegebenenfalls am Kohlenwasserstoffrest hydroxilierte Amine, wie Methylamin, Ethylamin, Propylamin, Isopropylamin, die vier isomeren Butylamine, Dimethylamin, Diethylamin, Dipropylamin, Diisopropylamin, Di-n-Butylamin, Pyrrolidin, Piperidin, Morpholin, Trimethylamin, Triethylamin, Tripropylamin, Chinuclidin, Pyridin, Chinolin, Isochinolin sowie Methanolamin, Ethanolamin, Propanolamin, Dimethanolamin, Diethanolamin oder Triethanolamin.

Beispiele für quartäre Ammoniumbasen sind Tetraalkylammoniumkationen, in denen die Alkylreste unabhängig voneinander geradkettige oder verzweigte ($C_1$-$C_6$)-Alkylgruppen sind, wie das Tetramethylammoniumkation, das Tetraethylammoniumkation oder das Trimethylethylammoniumkation, sowie weiterhin das Trimethylbenzylammoniumkation, das Triethylbenzylammoniumkation und das Trimethyl-2-hydroxyethylammoniumkation.

Besonders bevorzugt als Salzbildner sind das Ammoniumkation und Di- sowie Trialkylammoniumkationen, in denen die Alkylreste unabhängig voneinander geradkettige oder verzweigte, gegebenenfalls durch eine Hydroxylgruppe substituierte ($C_1$-$C_6$)-Alkylgruppen darstellen, wie beispielsweise das Dimethylammoniumkation, das Trimethylammoniumkation, das Triethylammoniumkation, das Di-(2-hydroxyethyl)-ammoniumkation und das Tri-(2-hydroxyethyl)-ammoniumkation.

Bevorzugte Safener sind Verbindungen der Formel (I), worin

A ($C_1$-$C_4$)-Alkandiyl oder ($C_3$-$C_6$)-Alkendiyl, wie $CH_2$, $CH(CH_3)$, $CH_2CH_2$, $CH_2CH_2CH_2$ oder $C(CH_3)_2$,

$R^*$ jeweils unabhängig von den anderen Resten $R^*$ Wasserstoff, Halogen, Halogen-($C_1$-$C_6$)-alkyl, Halogen-($C_1$-$C_6$)-alkoxy, ($C_1$-$C_8$)-Alkyl, ($C_1$-$C_8$)-Alkoxy, Nitro, Amino oder Amino, das durch ein oder zwei Reste aus der Gruppe ($C_1$-$C_4$)-alkyl und Acyl mit 1 bis 10 C-Atomen substituiert ist, oder Cyano, ($C_1$-$C_6$)-Alkylthio oder ($C_1$-$C_8$)-Alkylsulfonyl, vorzugsweise Wasserstoff, Halogen, Trifluormethyl, 2-Chlorethyl, Difluormethoxy, ($C_1$-$C_4$)-Alkyl, ($C_1$-$C_4$)-Alkoxy, ($C_1$-$C_4$)-Alkylthio, ($C_1$-$C_4$)-Alkylsulfonyl, Nitro, Amino, Mono-($C_1$-$C_4$)-alkylamino, Di-($C_1$-$C_4$)-alkylamino, ($C_1$-$C_4$)-Alkanoylamino, N-($C_1$-$C_4$)-Alkanoyl-N-($C_1$-$C_4$)-alkylamino, Benzoylamino, Benzyloxycarbonylamino, oder Cyano, insbesondere Wasserstoff,

$R^1$ Wasserstoff, ($C_1$-$C_{12}$)-Alkyl, ($C_3$-$C_8$)-Cycloalkyl, ($C_3$-$C_8$)-Heterocyclyl, ($C_2$-$C_{12}$)-Alkenyl, ($C_2$-$C_8$)-Alkinyl oder Phenyl,

wobei jeder der vorstehenden C-haltigen Reste unsubstituiert oder durch einen oder mehrere, vorzugsweise durch höchstens drei gleiche oder verschiedene Substituenten aus

der Gruppe Halogen, Halogen-($C_1$-$C_8$)-alkoxy, Nitro, Cyano, Hydroxy, ($C_1$-$C_8$)-Alkoxy, worin eine oder mehrere nicht nebeneinanderliegende $CH_2$-Gruppen durch Sauerstoff ersetzt sein können, ($C_1$-$C_8$)-Alkyl und Halo-($C_1$-$C_8$)-alkyl, letzte beiden nur bei cyclischen Resten, ($C_1$-$C_8$)-Alkylthio, ($C_1$-$C_6$)-Alkylsulfinyl, ($C_1$-$C_6$)-Alkylsulfonyl, ($C_2$-$C_8$)-Alkenylthio, ($C_2$-$C_8$)-Alkinylthio, ($C_2$-$C_8$)-Alkenyloxy, ($C_2$-$C_8$)-Alkinyloxy, ($C_3$-$C_7$)-Cycloalkyl, ($C_3$-$C_7$)-Cycloalkoxy, ($C_1$-$C_8$)-Alkoxycarbonyl, ($C_2$-$C_8$)-Alkenyloxycarbonyl, ($C_2$-$C_8$)-Alkinyloxycarbonyl, ($C_1$-$C_8$)-Alkylthiocarbonyl, ($C_1$-$C_8$)-Alkylcarbonyl, ($C_2$-$C_8$)-Alkenylcarbonyl, ($C_2$-$C_8$)-Alkinylcarbonyl, Hydroxyimino, ($C_1$-$C_4$)-Alkylimino, ($C_1$-$C_4$)-Alkoxyimino, ($C_1$-$C_8$)-Alkylcarbonylamino, ($C_2$-$C_8$)-Alkenylcarbonylamino, ($C_2$-$C_8$)-Alkinylcarbonylamino, Carbamoyl, ($C_1$-$C_8$)-Alkylcarbamoyl, Di-($C_1$-$C_6$)-Alkylcarbamoyl, ($C_2$-$C_6$)-Alkenylcarbamoyl, ($C_2$-$C_6$)-Alkinylcarbamoyl-($C_1$-$C_8$)-Alkoxycarbonylamino, ($C_1$-$C_8$)-Alkyl-amino-carbonylamino, ($C_1$-$C_8$)-Alkoxycarbonyloxy, ($C_1$-$C_8$)-Alkylcarbonyloxy, das unsubstituiert oder durch Halogen, Nitro, ($C_1$-$C_4$)-Alkoxy oder gegebenenfalls substituiertes Phenyl substituiert ist, ($C_2$-$C_6$)-Alkenylcarbonyloxy, ($C_2$-$C_6$)-Alkinylcarbonyloxy, Phenyl, Phenyl-($C_1$-$C_6$)-alkoxy, Phenyl-($C_1$-$C_6$)-alkoxycarbonyl, Phenoxy, Phenoxy-($C_1$-$C_6$)-alkoxy, Phenoxycarbonyl, Phenoxy-($C_1$-$C_6$)-alkoxycarbonyl, Phenylcarbonyloxy, Phenylcarbonylamino, Phenyl-($C_1$-$C_6$)-alkylcarbonylamino, wobei die letztgenannten 10 Reste im Phenylring unsubstituiert oder ein- oder mehrfach, vorzugsweise bis zu dreifach durch gleiche oder verschiedene Reste aus der Gruppe Halogen, ($C_1$-$C_4$)-Alkyl, ($C_1$-$C_4$)-Alkoxy, ($C_1$-$C_4$)-Halogenalkyl, ($C_1$-$C_4$)-Halogenalkoxy und Nitro substituiert sind, und Reste der Formel $-SiR^2R^3R^4$, $-O-SiR^2R^3R^4$, $R^2R^3R^4Si$-($C_1$-$C_6$)-alkoxy, $-CO-O-NR^2R^3$, $-O-N = CR^2R^3$, $-N = CR^2R^3$, $-NR^2R^3$, $-CONR^2R^3$, $-OCONR^2R^3$, $-O-(CH_2)_m-CH(OR^2)OR^3$, R'O-CHR''-CH(OR')-($C_1$-$C_6$)-alkoxy und einen fünf- bis sechsgliedrigen, gegebenenfalls benzokondensierten und gegebenenfalls substituierten, vorzugsweise bis zu dreifach durch Halogen und/oder ($C_1$-$C_4$)-Alkyl substituierten gesättigten oder ungesättigten heterocyclischen Rest mit bis zu drei gleichen oder verschiedenen Heteroatomen aus der Reihe S, O und N
substituiert ist,

R' unabhängig voneinander ($C_1$-$C_4$)-Alkyl oder paarweise zusammen einen ($C_1$-$C_6$)-Alkandiylrest,

R'' Wasserstoff oder ($C_1$-$C_4$)-Alkyl,

$R^2$ und $R^3$ unabhängig voneinander Wasserstoff, ($C_1$-$C_4$)-Alkyl oder gegebenenfalls substituiertes Phenyl oder gemeinsam eine unverzweigte ($C_2$-$C_4$)-Alkandiylkette und

m eine ganze Zahl von 0 bis 6 bedeuten.

Weiter bevorzugt sind als Safener Verbindungen der Formel (I), worin

R* wie oben definiert ist und mindestens zwei Reste R* am Benzolring Wasserstoff,

$R^1$ Wasserstoff, ($C_1$-$C_{12}$)-Alkyl, ($C_3$-$C_8$)-Cycloalkyl, ($C_3$-$C_8$)-Heterocyclyl, ($C_2$-$C_{12}$)-Alkenyl, ($C_2$-$C_8$)-Alkinyl,

wobei jeder der vorstehenden C-haltigen Reste unsubstituiert oder durch einen oder mehrere, vorzugsweise durch höchstens zwei, insbesondere höchstens einen Substituenten aus der Gruppe enthaltend Hydroxy, ($C_1$-$C_8$)-Alkoxy, Alkoxy-polyalkylenoxy mit insgesamt 2 bis 8 C-Atomen, ($C_1$-$C_4$)-Alkylthio, ($C_2$-$C_4$)-Alkenylthio, ($C_2$-$C_4$)-Alkinylthio, ($C_2$-$C_4$)-Alkenyloxy, ($C_2$-$C_4$)-Alkinyloxy, Mono- und Di-($C_1$-$C_2$)-alkylamino, ($C_1$-$C_4$)-Alkoxycarbonyl, ($C_2$-$C_4$)-Alkenyloxycarbonyl, ($C_2$-$C_4$)-Alkinyloxycarbonyl, ($C_1$-$C_4$)-Alkylcarbonyl, ($C_2$-$C_4$)-Alkenylcarbonyl, ($C_2$-$C_4$)-Alkinylcarbonyl, ($C_1$-$C_4$)-Alkylcarbonylamino, ($C_2$-$C_4$)-Alkenylcarbonylamino, Carbamoyl, ($C_1$-$C_8$)-Alkylcarbamoyl, Di-($C_1$-$C_6$)-Alkylcarbamoyl, ($C_1$-$C_4$)-Alkoxycarbonyloxy, ($C_1$-$C_4$)-Alkylcarbonyloxy, das unsubstituiert oder durch ein oder zwei Reste aus der Gruppe Halogen und ($C_1$-$C_4$)-Alkoxy substituiert ist, ($C_2$-$C_4$)-Alkenylcarbonyloxy, ($C_2$-$C_4$)-Alkinylcarbonyloxy, Phenyl, Phenyl-($C_1$-$C_4$)-alkoxy, Phenyl-($C_1$-$C_4$)-alkoxycarbonyl, Phenoxy, Phenoxy-($C_1$-$C_4$)-alkoxy, Phenoxycarbonyl, Phenoxy-($C_1$-$C_4$)-alkoxycarbonyl, Phenylcarbonyloxy, wobei die letztgenannten 8 Reste im Phenylring unsubstituiert oder durch einen oder mehrere Reste aus der Gruppe enthaltend Halogen, ($C_1$-$C_2$)-Alkyl, ($C_1$-$C_2$)-Alkoxy, ($C_1$-$C_2$)-Halogenalkyl, ($C_1$-$C_2$)-Halogenalkoxy und Nitro substituiert sind, und Reste der Formel $-SiR^2R^3R^4$, $-O-SiR^2R^3R^4$, $R^2R^3R^4Si$-($C_1$-$C_4$)-alkoxy, $-O-N = CR^2R^3$, $-N = CR^2R^3$, $O-(CH_2)_m-CH(OR^2)OR^3$, R'O-CHR''-CH(OR')-($C_1$-$C_6$)-alkoxy, $NR^2R^3$, $-CONR^2R^3$ und $-OCONR^2R^3$
substituiert ist,

R' unabhängig voneinander ($C_1$-$C_4$)-Alkyl oder paarweise zusammen einen ($C_1$-$C_6$)-Alkandiylrest und

| | |
|---|---|
| R'' | Wasserstoff oder ($C_1$-$C_4$)-Alkyl, |
| $R^2$ und $R^3$ | gleich oder verschieden sind und unabhängig voneinander Wasserstoff, ($C_1$-$C_4$)-Alkyl oder gegebenenfalls substituiertes Phenyl, oder gemeinsam eine gegebenenfalls substituierte ($C_2$-$C_4$)-Alkandiylkette, |
| $R^5$ und $R^6$ | gleich oder verschieden sind und unabhängig voneinander Wasserstoff oder ($C_1$-$C_4$)-Alkyl, |
| $R^7$ und $R^8$ | gleich oder verschieden sind und unabhängig voneinanderWasserstoff oder ($C_1$-$C_4$)-Alkyl, |
| $R^9$ und $R^{10}$ | unabhängig voneinander Wasserstoff oder ($C_1$-$C_4$)-Alkyl und |
| m | eine ganze Zahl von 0 bis 2 |

bedeuten.

Bevorzugt sind erfindungsgemäße Safener der Formel (I) in denen die Gruppe $B^1$-$A(B^2)_n$-O- in $\alpha$-Stellung zu einem Brückenkopfatom des Bicyclus steht, wobei das Brückenkopfatom mit einem N-Atom aus der Gruppe $W^1$ = N oder $W^2$ = N = N oder N = CR* verbunden ist.

Bevorzugt sind vor allem Safener auf Basis der bicyclischen Systeme Benzthiazol, Benzoxazol, Chinoxalin, aber auch Benzimidazol, Cinnolin, Chinazolin und Benzo-1,2,4-triazin, insbesondere folgende Verbindungen der Formeln (Ia) bis (Ih)

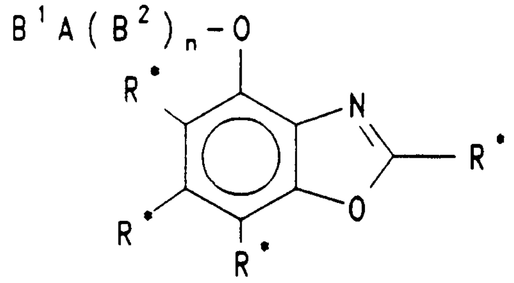

(Ia)

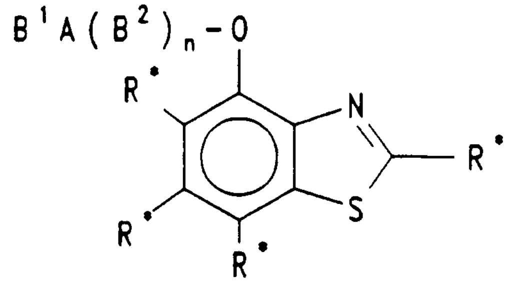

(Ib)

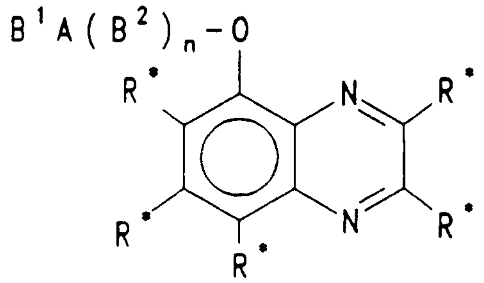

(Ic)

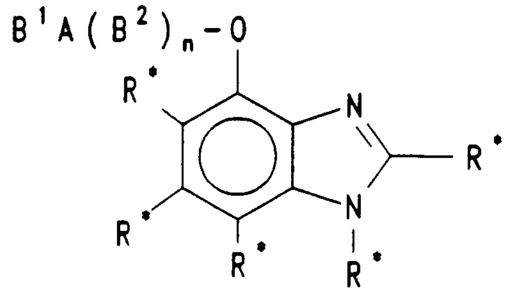

(Id)

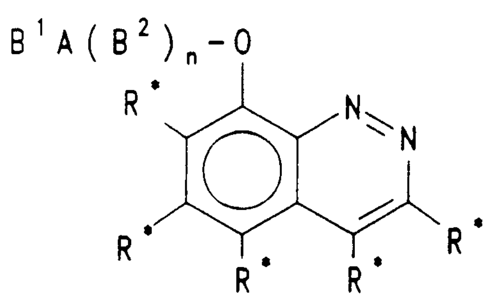

(Ie)

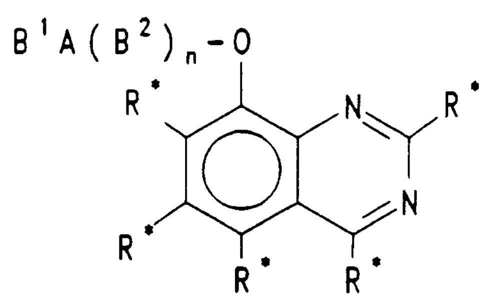

(If)

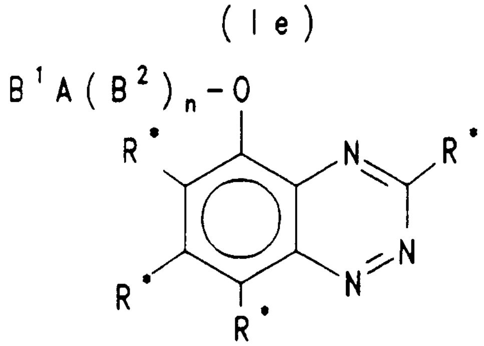

(Ig)

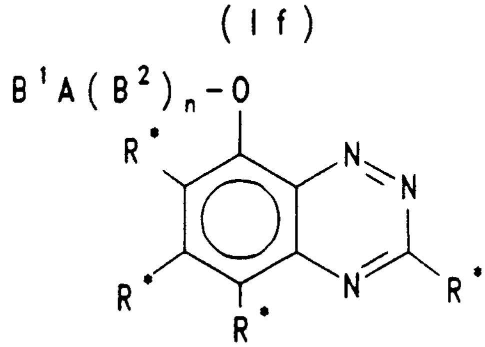

(Ih)

Besonders bevorzugt sind Safener der Formeln (Ia), (Ib), (Ic), worin n = 0 oder 1 und $B^1$ und $B^2$ aus der Gruppe der Carbonsäureester ist.

Einige Verbindungen der Formel (I) sind bereits bekannt bzw. einige der betreffenden Strukturklassen sind in allgemeiner Form beschrieben worden; vgl. z. B. Hawaiian Sugar Technologists, Rept. 24, 152 (1965); Symp. Soc. Exptl. Biol. 17, 98 (1963); JP-A-04-028893 (1992), JP-A-02-306916 (1990). Die

Safenerwirkung der Verbindungen (I) war jedoch bisher nicht bekannt.

Gegenstand der Erfindung sind somit auch die chemischen Verbindungen der obengenannten Formel (I) und deren Salze, worin A, $B^1$, $B^2$, n, $R^*$, $W^1$ und $W^2$ wie oben definiert sind, ausgenommen solche Verbindungen (I), die bereits bekannt sind.

Die Verbindungen der Formel (I) lassen sich nach oder analog allgemein bekannten Verfahren herstellen (Brettle, J. Chem. Soc. (1956) 1891; Eckstein, Roczniki Chem. 30 (1956) 633; US 2 697 708; Newman et al., J. Am. Chem. Soc. 69 (1947) 718; M. P. Cava, N. K. Bhattacharyya, J. Org. Chem. 23 (1958) 1614; D. Heilmann, G. Kempter, Wiss. Z. Pädagog. Hochsch. "Karl Liebknecht", Postdam 25 (1981) 35; Ger 1 099 544; US 3 010 962).

So kann die Herstellung der erfindungsgemäßen Verbindungen der Formel (I) in der Weise erfolgen, daß man

a) eine Verbindung der Formel

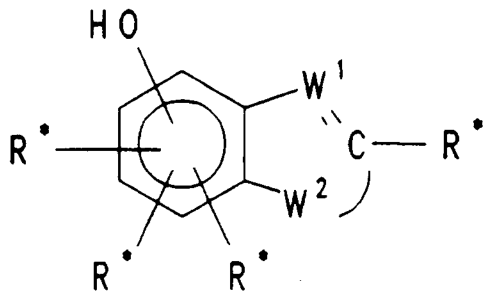

(II)

mit einer Verbindung der Formel (III),

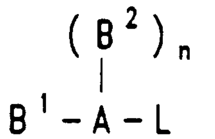

(III)

worin n eine Abgangsgruppe bedeutet, umsetzt oder

b) im Falle n = 0 und $B^1$ = Derivat der Carboxygruppe, eine Verbindung der Formel (IV)

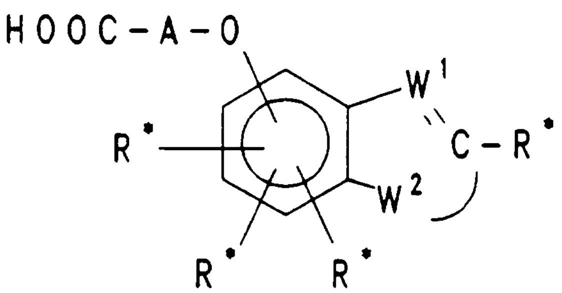

(IV)

zu Säurederivaten der Formel (I), n = 0 umsetzt, vorzugsweise mit Alkoholen $R^1$-OH, Thioalkoholen $R^1SH$, Aminen $NR^1R^4$ umsetzt oder amidiert,

c) im Falle, daß $B^1$ und $B^2$ Carbonsäurederivatgruppen sind eine Verbindung der Formel (I')

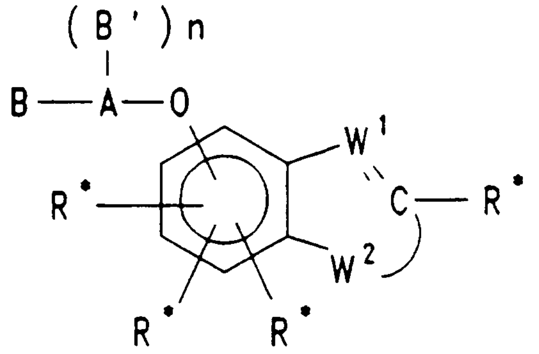

(I ')

worin B und B' analog B' und $B^2$ definiert sind, zu Verbindungen der Formel (I) umestert bzw. amidiert, wobei in den Formeln (II), (III), (IV) und (I') die Reste A, $B^1$, $B^2$, n, $R^*$, $W^1$ und $W^2$ wie in Formel (I) definiert sind.

Die Umsetzungen nach Variante a) erfolgen vorzugsweise in dipolar aprotischen Lösungsmitteln wie Dimethylsulfoxid, N,N-Dimethylformamid, Diethylether oder Aceton in Gegenwart einer Base wie Alkalialkoholat, Alkalihydrid oder Alkalicarbonate, z. B. Kaliumcarbonat. Die optimalen Temperaturbedingungen der Umsetzungen hängen auch vom Lösungsmittel ab und können in Vorversuchen bestimmt werden. Bei Reaktionen in Dimethylsulfoxid oder N,N-Dimethylformamid hat sich häufig leicht erhöhte Temperatur, beispielsweise zwischen 50 und 80 °C bewährt.

Die Umsetzungen nach Variante b) werden entweder unter Säurekatalyse, wobei vorzugsweise Schwefelsäure Verwendung findet, oder in Gegenwart eines die Carboxylgruppe aktivierenden Reagenzes, wie z. B. Thionylchlorid, Dicyclohexylcarbodiimid oder N,N'-Carbonyldiimidazol in dipolar aprotischen Lösungsmitteln oder Halogenkohlenwasserstoffen, wie z. B. Chloroform oder Tetrachlormethan bei Temperaturen von Raumtemperatur bis zum Siedepunkt des Reaktionsgemisches, insbesondere bei Rückflußtemperatur durchgeführt.

Die Umesterungen bzw. Amidierungen nach Variante c) erfolgen vornehmlich in der Weise, daß eine Verbindung der Formel (V) in Gegenwart von Titanalkoholaten als Katalysator mit den Alkoholen bzw. den Aminen bei erhöhten Temperaturen, insbesondere bei Rückflußtemperatur des Reaktionsgemisches umgesetzt wird.

Die Verbindungen der Formel (II) sind bekannt oder lassen sich nach oder analog allgemein bekannten Verfahreen herstellen (J. H. Musser, J. Med. Chem. 30 (1987) 62, z. B. für Benzoxazole; A. Albert, A. Hampton J. Chem. Soc. 4985 (1952), z.B. für Chinoxaline, Cinnoline und Chinazoline; U. Holstein, G. E. Krisov, Org. Magn. Reson. 14 (1980) 300, z. B. für Chinoxaline; C. F. H. Allen, J. Van Allan, Org. Synth. Coll. Vol. 3, 76, z. B. für Benzthiazole; E. C. Wagner, M. H. Millett, Org. Synth. Coll. Vol. 2, 65, z. B. für Benzimidazole; H. Neuhoefer in Comprehensive Heterocyclic Chemistry 3, 430, z. B. für Benzotriazine; P. A. Grieco, J. Am. Chem. Soc. 99 (1977) 5773 und Organikum, 19. Aufl. 1993, z. B. für Umwandlungen von funktionellen Gruppen.

Werden die erfindungsgemäßen Verbindungen der Formel (I) in subtoxischen Konzentrationen zusammen mit den herbiziden Wirkstoffen oder auch in einer beliebigen Reihenfolge ausgebracht, so sind sie in der Lage, die phytotoxischen Nebenwirkungen dieser Herbizide zu reduzieren bzw. völlig aufzuheben, ohne jedoch die Wirksamkeit der Herbizide gegenüber den Schadpflanzen zu vermindern.

Hierdurch kann das Einsatzgebiet herkömmlicher Pflanzenschutzmittel ganz erheblich erweitert werden. Herbizide, deren phytotoxische Nebenwirkungen auf Kulturpflanzen mittels Verbindungen der Formel (I) herabgesetzt werden können, sind z. B. Carbamate, Thiocarbamate, Halogenacetanilide, substituierte Phenoxy-, Naphthoxy- und Phenoxy-phenoxycarbonsäurederivate sowie Heteroaryloxy-phenoxyalkancarbonsäurederivate, wie Chinolyloxy-, Chinoxalyloxy-, Pyridyloxy-, Benzoxalyloxy- und Benzthiazolyloxy-phenoxyalkancarbonsäureester, Cyclohexandionabkömmlinge, Imidazolinone, Pyrimidyloxy-pyridincarbonsäure-derivate, Pyrimidyloxy-benzoesäure-derivate, Sulfonylharnstoffe, Triazolopyrimidin-sulfonamid-derivate und S-(N-Aryl-N-alkylcarbamoylmethyl)-dithiophosphorsäureester. Bevorzugt sind dabei Phenoxyphenoxy- und Heteroaryloxy-phenoxycarbonsäureester und -salze, Sulfonylharnstoffe und Imidazolinone.

A) Herbizide vom Typ der Phenoxyphenoxy- und Heteroaryloxyphenoxycarbonsäure-($C_1$-$C_4$)alkyl-, ($C_2$-$C_4$)alkenyl- und ($C_3$-$C_4$)alkinylester wie

A1) Phenoxy-phenoxy- und Benzyloxy-phenoxy-carbonsäure-derivate, z. B.

2-(4-(2,4-Dichlorphenoxy)-phenoxy)-propionsäuremethylester (Diclofop-methyl),

2-(4-(4-Brom-2-chlorphenoxy)-phenoxy)-propionsäuremethylester (s. DE-A-2601548),
2-(4-(4-Brom-2-fluorphenoxy)-phenoxy)-propionsäuremethylester (s. US-A-4808750),
2-(4-(2-Chlor-4-trifluormethylphenoxy)-phenoxy)-propionsäuremethylester (s. DE-A-2433067),
2-(4-(2-Fluor-4-trifluormethylphenoxy)-phenoxy)-propionsäuremethylester (s. US-A-4808750),
2-(4-(2,4-Dichlorbenzyl)-phenoxy)propionsäuremethylester (s. DE-A-2417487),
4-(4-(4-Trifluormethylphenoxy)-phenoxy)-pent-2-en-säureethylester,
2-(4-(4-Trifluormethylphenoxy)-phenoxy)-propionsäuremethylester (s. DE-A-2433067),
A2) "Einkernige" Heteroaryloxy-phenoxy-alkancarbonsäurederivate, z. B.
2-(4-(3,5-Dichlorpyridyl-2-oxy)-phenoxy)-propionsäureethylester (s. EP-A-2925),
2-(4-(3,5-Dichlorpyridyl-2-oxy)-phenoxy)-propionsäurepropargylester (EP-A-3114),
2-(4-(3-Chlor-5-trifluormethyl-2-pyridyloxy)-phenoxy-propionsäure-methylester (s. EP-A-3890),
2-(4-(3-Chlor-5-trifluormethyl-2-pyridyloxy)-phenoxy)-propionsäure-ethylester (s. EP-A-3890),
2-(4-(5-Chlor-3-fluor-2-pyridyloxy)-phenoxy)-propionsäurepropargylester (EP-A-191736),
2-(4-(5-Trifluormethyl-2-pyridyloxy)-phenoxy)-propionsäurebutylester (Fluazifopbutyl),
A3) "Zweikernige" Heteroaryloxy-phenoxy-alkancarbonsäurederivate, z. B.
2-(4-(6-Chlor-2-chinoxalyloxy)-phenoxy)-propionsäuremethylester und -ethylester (Quizalofop-methyl und -ethyl),
2-(4-(6-Fluor-2-chinoxalyloxy)-phenoxy)-propionsäuremethylester (s. J. Pest. Sci. Vol. 10, 61 (1985)),
2-(4-(6-Chlor-2-chinoxalyloxy)-phenoxy)-propionsäure und -2-isopropylidenaminooxyethylester (Propaquizafop u. Ester),
2-(4-(6-Chlorbenzoxazol-2-yl-oxy)-phenoxy)-propionsäureethylester (Fenoxapropethyl), dessen D(+) Isomer (Fenoxaprop-P-ethyl) und
2-(4-(6-Chlorbenzthiazol-2-yloxy)-phenoxypropionsäureethylester (s. DE-A-2640730),
2-(4-(6-Chlorchinoxalyloxy)-phenoxy-propionsäure-tetrahydrofur-2-ylmethyl-ester (s. EP-A 323 727),

B) Herbizide aus der Sulfonylharnstoff-Reihe, wie z. B. Pyrimidin- oder Triazinylaminocarbonyl-[benzol-, pyridin-, pyrazol-, thiophen- und (alkylsulfonyl)alkylamino-]-sulfamide. Bevorzugt als Substituenten am Pyrimidinring oder Triazinring sind Alkoxy, Alkyl, Haloalkoxy, Haloalkyl, Halogen oder Diamethylamino, wobei alle Substituenten unabhängig voneinander kombinierbar sind. Bevorzugte Substituenten im Benzol-, Pyridin-, Pyrazol-, Thiophen- oder (Alkylsulfonyl)alkylamino-Teil sind Alkyl, Alkoxy, Halogen, Nitro, Alkoxycarbonyl, Aminocarbonyl, Alkylaminocarbonyl, Dialkylaminocarbonyl, Alkoxyaminocarbonyl, Alkyl, Alkocyaminocarbonyl, Haloalkoxy, Haloalkyl, Alkylcarbonyl, Alkoxyalkyl, (Alkansulfonyl)alkylamino. Geeignete Sulfonylharnstoffe sind beispielsweise

B1) Phenyl- und Benzylsulfonylharnstoffe und verwandte Verbindungen, z. B.
1-(2-Chlorphenylsulfonyl)-3-(4-methoxy-6-methyl-1,3,5-triazin-2-yl)harnstoff (Chlorsulfuron),
1-(2-Ethoxycarbonylphenylsulfonyl)-3-(4-chlor-6-methoxypyrimidin-2-yl)harnstoff (Chlorimuron-ethyl),
1-(2-Methoxyphenylsulfonyl)-3-(4-methoxy-6-methyl-1,3,5-triazin-2-yl)harnstoff (Metsulfuron-methyl),
1-(2-Chlorethoxy-phenylsulfonyl)-3-(4-methoxy-6-methyl-1,3,5-triazin-2-yl)harnstoff (Triasulfuron),
1-(2-Methoxycarbonyl-phenylsulfonyl)-3-(4,6-dimethyl-pyrimidin-2-yl)harnstoff (Sulfometuron-methyl),
1-(2-Methoxycarbonylphenylsulfonyl)-3-(4-methoxy-6-methyl-1,3,5-triazin-2-yl)-3-methylharnstoff (Tribenuron-methyl),
1-(2-Methoxycarbonylbenzylsulfonyl)-3-(4,6-dimethoxy-pyrimidin-2-yl)harnstoff (Bensulfuron-methyl),
1-(2-Methoxycarbonylphenylsulfonyl)-3-(4,6-bis-(difluormethoxy)pyrimidin-2-yl)harnstoff (Primisulfuron-methyl),
3-(4-Ethyl-6-methoxy-1,3,5-triazin-2-yl)-1-(2,3-dihydro-1,1-dioxo-2-methylbenzo[b]thiophen-7-sulfonyl)-harnstoff (s. EP-A-79683),
3-(4-Ethoxy-6-ethyl-1,3,5-triazin-2-yl)-1-(2,3-dihydro-1,1-dioxo-2-methylbenzo[b]thiophen-7-sulfonyl)-harnstoff (s. EP-A-79683),
4-Iod-2-[3-(4-methoxy-6-methyl-1,3,5-triazinyl)-ureido-sulfonyl]-benzoesäuremethylester, Natriumsalz (s. WO-A-92/13845)
B2) Thienylsulfonylharnstoffe, z. B. 1-(2-Methoxycarbonylthiophen-3-yl)-3-(4-methoxy-6-methyl-1,3,5-triazin-2-yl)harnstoff (Thifensulfuron-methyl),
B3) Pyrazolylsulfonylharnstoffe, z. B.
1-(4-Ethoxycarbonyl-1-methylpyrazol-5-yl-sulfonyl)-3-(4,6-dimethoxypyrimidin-2-yl)harnstoff (Pyrazosulfuron-methyl),
3-Chlor-5-(4,6-dimethoxypyrimidin-2-ylcarbamoylsulfamoyl)-1-methyl-pyrazol-4-carbonsäuremethylester (s. EP 282613),
5-(4,6-Dimethylpyrimidin-2-yl-carbamoylsulfamoyl)-1-(2-pyridyl)-pyrazol-4-carbonsäuremethylester (NC-330, s. Brighton Crop Prot. Conference - Weeds - 1991, Vol. 1, 45 ff.),

B4) Sulfondiamid-Derivate, z. B.
3-(4,6-Dimethoxypyrimidin-2-yl)-1-(N-methyl-N-methylsulfonylaminosulfonyl)harnstoff (Amidosulfuron) und Strukturanaloge (s. EP-A-0131258 und Z. Pfl. Krankh. Pfl. Schutz 1990, Sonderheft XII, 489-497),
B5) Pyridylsulfonylharnstoffe, z. B.
1-(3-N,N-Dimethylaminocarbonylpyridin-2-yl-sulfonyl)-3-(4,6-dimethoxypyrimidin-2-yl)harnstoff (Nicosulfuron),
1-(3-Ethylsulfonylpyridin-2-yl-sulfonyl)-3-(4,6-dimethoxy-pyrimidin-2-yl)harnstoff (DPX-E 9636, s. Brighton Crop Prot. Conf. - Weeds - 1989, S. 23 ff.),
Pyridylsulfonylharnstoffe, wie sie in DE-A-4000503 und DE-A-4030577 beschrieben sind, vorzugsweise solche der Formel

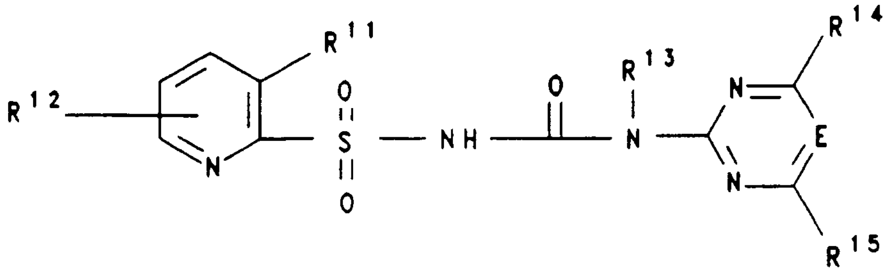

worin

| | |
|---|---|
| E | CH oder N vorzugsweise CH, |
| $R^{11}$ | Iod oder $NR^{16}R^{17}$, |
| $R^{12}$ | H, Halogen, Cyano, $(C_1$-$C_3)$-Alkyl, $(C_1$-$C_3)$-Alkoxy, $(C_1$-$C_3)$-Haloalkyl, $(C_1$-$C_3)$-Haloalkoxy, $(C_1$-$C_3)$-Alkylthio, $(C_1$-$C_3)$-Alkoxy-$(C_1$-$C_3)$-alkyl, $(C_1$-$C_3)$-Alkoxy-carbonyl, Mono- oder Di-$(C_1$-$C_3)$-alkyl-amino, $(C_1$-$C_3)$-Alkyl-sulfinyl oder -sulfonyl, $SO_2$-$NR^aR^b$ oder CO-$NR^aR^b$, insbesondere H, |
| $R^a$,$R^b$ | unabhängig voneinander H, $(C_1$-$C_3)$-Alkyl, $(C_1$-$C_3)$-Alkenyl, $(C_1$-$C_3)$-Alkinyl oder zusammen $-(CH_2)_4-$, $-(CH_2)_5-$ oder $(CH_2)_2$-O-$(CH_2)_2$-, |
| $R^{13}$ | H oder $CH_3$, |
| $R^{14}$ | Halogen, $(C_1$-$C_2)$-Alkyl, $(C_1$-$C_2)$-Alkoxy, $(C_1$-$C_2)$-Haloalkyl, vorzugsweise $CF_3$, $(C_1$-$C_2)$-Haloalkoxy, vorzugsweise $OCHF_2$ oder $OCH_2CF_3$, |
| $R^{15}$ | $(C_1$-$C_2)$-Alkyl, $(C_1$-$C_2)$-Haloalkoxy, vorzugsweise $OCHF_2$, oder $(C_1$-$C_2)$-Alkoxy, und |
| $R^{16}$ | $(C_1$-$C_4)$-Alkyl und |
| $R^{17}$ | $(C_1$-$C_4)$-Alkylsulfonyl oder |
| $R^{16}$ und $R^{17}$ | gemeinsam eine Kette der Formel $-(CH_2)_3SO_2-$ oder $-(CH_2)_4SO_2$ bedeuten, |

z. B. 3-(4,6-Dimethoxypyrimidin-2-yl)-1-(3-N-methylsulfonyl-N-methylaminopyridin-2-yl)-sulfonylharnstoff, oder deren Salze,
B6) Alkoxyphenoxysulfonylharnstoffe, wie sie in EP-A-0342569 beschrieben sind, vorzugsweise solche der Formel

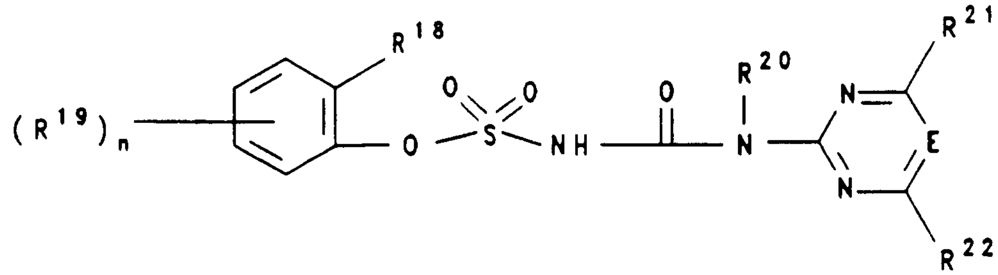

worin

| | |
|---|---|
| E | CH oder N, vorzugsweise CH, |
| $R^{18}$ | Ethoxy, Propoxy oder Isopropoxy, |
| $R^{19}$ | Wasserstoff, Halogen, $NO_2$, $CF_3$, CN, $(C_1$-$C_4)$-Alkyl, $(C_1$-$C_4)$-Alkoxy, $(C_1$-$C_4)$-Alkylthio |

oder ($C_1$-$C_3$)-Alkoxy-carbonyl, vorzugsweise in 6-Position am Phenylring,

n 1, 2 oder 3, vorzugsweise 1,

$R^{20}$ Wasserstoff, ($C_1$-$C_4$)-Alkyl oder ($C_3$-$C_4$)-Alkenyl,

$R^{21}$,$R^{22}$ unabhängig voneinander Halogen, ($C_1$-$C_2$)-Alkyl, ($C_1$-$C_2$)-Alkoxy, ($C_1$-$C_2$)-Haloalkyl, ($C_1$-$C_2$)-Haloalkoxy oder ($C_1$-$C_2$)-Alkoxy-($C_1$-$C_2$)-alkyl, vorzugsweise $OCH_3$ oder $CH_3$, bedeuten, z. B. 3-(4,6-Dimethoxypyrimidin-2-yl)-1-(2-ethoxyphenoxy)-sulfonylharnstoff, oder deren Salze,

und andere verwandte Sulfonylharnstoffderivate und Mischungen daraus,

C) Chloracetanilid-Herbizide wie
N-Methoxymethyl-2,6-diethyl-chloracetanilid (Alachlor),
N-(3'-Methoxyprop-2'-yl)-2-methyl-6-ethyl-chloracetanilid (Metolachlor),
N-(3-Methyl-1,2,4-oxdiazol-5-yl-methyl)-chloressigsäure-2,6-dimethylanilid,
N-(2,6-Dimethylphenyl)-N-(1-pyrazolylmethyl)-chloressigsäureamid (Metazachlor),

D) Thiocarbamate wie
S-Ethyl-N,N-dipropylthiocarbamat (EPTC) oder
S-Ethyl-N,N-diisobutylthiocarbamat (Butylate),

E) Cyclohexandion-Derivate wie
3-(1-Allyloxyiminobutyl)-4-hydroxy-6,6-dimethyl-2- oxocyclohex-3-encarbonsäuremethylester (Alloxydim),
2-(1-Ethoximinobutyl)-5-(2-ethylthiopropyl)-3-hydroxy-cyclohex-2-en-1-on (Sethoxydim),
2-(1-Ethoximinobutyl)-5-(2-phenylthiopropyl)-3-hydroxy-cyclohex-2-en-1-on (Cloproxydim),
2-(1-(3-Chlorallyloxy)iminobutyl)-5-[2-(ethylthio)propyl]-3-hydroxy-cyclohex-2-en-1-on,
2-(1-(3-Chlorallyloxy)iminopropyl)-5-[2-(ethylthio)propyl]-3-hydroxy-cyclohex-2-en-1-on (Clethodim),
2-(1-(Ethoxyimino)-butyl)-3-hydroxy-5-(thian-3-yl)-cyclohex-2-enon (Cycloxydim),
oder
2-(1-Ethoxyiminopropyl)-5-(2,4,6-trimethylphenyl)-3-hydroxy-cyclohex-2-en-1-on (Tralkoxydim),

F) 2-(4-Alkyl-5-oxo-2-imidazolin-2-yl)-benzoesäurederivate oder 2-(4-Alkyl-5-oxo-2-imidazolin-2yl)-heteroarylcarbonsäurederivate wie z. B.
2-(4-Isopropyl-4-methyl-5-oxo-2-imidazolin-2-yl)-5-methylbenzoesäuremethylester und
2-(4-Isopropyl-4-methyl-5-oxo-2-imidazolin-2-yl)-4-methylbenzoesäure (Imazamethabenz),
5-Ethyl-2-(4-isopropyl-4-methyl-5-oxo-2-imidazolin-2-yl)-pyridin-3-carbonsäure (Imazethapyr),
2-(4-Isopropyl-4-methyl-5-oxo-2-imidazolin-2-yl)-chinolin-3-carbonsäure (Imazaquin),
2-(4-Isopropyl-4-methyl-5-oxo-2-imidazolin-2-yl)-pyridin-3-carbonsäure (Imazapyr),
5-Methyl-2-(4-Isopropyl-4-methyl-5-oxo-2-imidazolin-2yl)-pyridin-3-carbonsäure (Imazethamethapyr),

G) Triazolopyrimidinsulfonamidderivate, z. B.
N-(2,6-Difluorphenyl)-7-methyl-1,2,4-triazolo-(1,5-c)-pyrimidin-2-sulfonamid (Flumetsulam),
N-(2,6-Dichlor-3-methylphenyl)-5,7-dimethoxy-1,2,4-triazolo-(1,5-c)-pyrimidin-2-sulfonamid,
N-(2,6-Difluorphenyl)-7-fluor-5-methoxy-1,2,4-triazolo-(1,5-c)-pyrimidin-2-sulfonamid
N-(2,6-Dichlor-3-methylphenyl)-7-chlor-5-methoxy-1,2,4-triazolo-(1,5- c)-pyrimidin-2- sulfonamid,
N-(2-Chlor-6-methoxycarbonyl)-5,7-dimethyl-1,2,4-triazolo-(1,5-c)-pyrimidin-2-sulfonamid
(siehe z. B. EP-A-343 752, US- 4 988 812),

H) Benzoylcyclohexandionderivate, z. B.
2-(2-Chlor-4-methylsulfonylbenzoyl)-cyclohexan-1,3-dion (SC-0051, s. EP-A-137963),
2-(2-Nitrobenzoyl)-4,4-dimethyl-cyclohexan-1,3-dion (s. EP-A-274634),
2-(2-Nitro-3-methylsulfonylbenzoyl)-4,4-dimethyl-cyclohexan-1,3-dion (s. WO-91/13548),

J) Pyrimidinyloxy-pyrimidincarbonsäure- bzw. Pyrimidinyloxy-benzoesäure-Derivate, z. B.
3-(4,6-Dimethoxypyrimidin-2-yl)-oxy-pyridin-2-carbonsäurebenzylester(EP-A-249 707),
3-(4,6-Dimethoxypyrimidin-2-yl)-oxy-pyridin-2-carbonsäuremethylester(EP-A-249 707),
2,6-Bis[(4,6-dimethoxypyrimidin-2-yl)-oxy]-benzoesäure (EP-A-321 846), 2,6-Bis[(4,6-dimethoxypyrimidin-2-yl)-oxy]-benzoesäure-(1-ethoxycarbonyloxyethyl)-ester (EP-A-472 113) und

K) S-(N-Aryl-N-alkyl-carbamoylmethyl)-dithiophosphorsäureester wie S-[N-(4-Chlorphenyl)-N-isopropylcarbamoylmethyl]-O,O-dimethyl-dithiophosphat (Anilofos).

Die obengenannten Herbizide der Gruppen A bis K sind dem Fachmann bekannt und in der Regel in "The Pesticide Manual", British Crop Protection Council, 9. Auflage 1991 oder 8. Auflage 1987 oder in "Agricultural Chemicals Book II, Herbicides", by W. T. Thompson, Thompson Publications, Fresno CA, USA 1990 oder in "Farm Chemicals Handbook '90", Meister Publishing Company, Willoughby OH, USA 1990 beschrieben. Imazethamethapyr ist aus Weed Techn. 1991, Vol. 5, 430-438 bekannt.

Die herbiziden Wirkstoffe und die erwähnten Safener können zusammen (als fertige Formulierung oder im Tank-mix-Verfahren) oder in beliebiger Reihenfolge nacheinander ausgebracht werden. Das Gewichtsverhältnis Safener:Herbizid kann innerhalb weiter Grenzen variieren und ist vorzugsweise im Bereich von 1:10 bis 10:1, insbesondere von 1:10 bis 5:1. Die jeweils optimalen Mengen an Herbizid und Safener sind vom Typ des verwendeten Herbizids oder vom verwendeten Safener sowie von der Art des zu behandelnden Pflanzenbestandes abhängig und lassen sich von Fall zu Fall durch entsprechende Vorversuche ermitteln.

Haupteinsatzgebiete für die Anwendung der Safener sind vor allem Getreidekulturen (Weizen, Roggen, Gerste, Hafer), Reis, Mais, Sorghum, aber auch Baumwolle und Sojabohne, vorzugsweise Getreide, Reis und Mais.

Die Safener der Formel (I) können je nach ihren Eigenschaften zur Vorbehandlung des Saatgutes der Kulturpflanze (Beizung der Samen) verwendet werden oder vor der Saat in die Saatfurchen eingebracht oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein. Bevorzugt ist die gemeinsame Anwendung mit dem Herbizid. Hierzu können Tankmischungen oder Fertigformulierungen eingesetzt werden.

Die benötigten Aufwandmengen der Safener können je nach Indikation und verwendetem Herbizid innerhalb weiter Grenzen schwanken und liegen in der Regel im Bereich von 0,001 bis 5 kg, vorzugsweise 0,005 bis 0,5 kg Wirkstoff je Hektar.

Gegenstand der vorliegenden Erfindung ist deshalb auch ein Verfahren zum Schutz von Kulturpflanzen vor phytotoxischen Nebenwirkungen von Herbiziden, das dadurch gekennzeichnet ist, daß eine wirksame Menge einer Verbindung der Formel (I) vor, nach oder gleichzeitig mit dem Herbizid auf die Pflanzen, Pflanzensamen oder die Anbaufläche appliziert wird.

Gegenstand der Erfindung sind auch pflanzenschützende Mittel, die einen Wirkstoff der Formel (I) und übliche Formulierungshilfsmittel enthalten, sowie herbizide Mittel, die einen Wirkstoff der Formel (I) und ein Herbizid sowie im Bereich des Pflanzenschutzes übliche Formulierungshilfsmittel enthalten.

Die Verbindungen der Formel (I) und deren Kombinationen mit einem oder mehreren der genannten Herbizide können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), emulgierbare Konzentrate (EC), wasserlösliche Pulver (SP), wasserlösliche Konzentrate (SL), konzentrierte Emulsionen (EW) wie Öl-in-Wasser und Wasser-in- Öl-Emulsionen, versprühbare Lösungen oder Emulsionen, Kapselsuspensionen (CS), Dispersionen auf Öl- oder Wasserbasis (SC), Suspoemulsionen, Suspensionskonzentrate, Stäubemittel (DP), ölmischbare Lösungen (OL), Beizmittel, Granulate (GR) in Form von Mikro-, Sprüh-, Aufzugs- und Adsorptionsgranulaten, Granulate für die Boden- bzw. Streuapplikation, wasserlösliche Granulate (SG), wasserdispergierbare Granulate (WG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie" Band 7, C. Hauser Verlag München, 4. Aufl. 1986; Wade van Valkenburg, "Pesticide Formulations", Marcel Dekker N.Y., 1973; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N. J.; H. v. Olphen, "Introduction to Clay Colloid Chemistry", 2nd Ed., J. Wiley & Sons, N. Y.; Marsden, "Solvents Guide", 2nd Ed., Interscience, N.Y. 1963; McCutcheon's "Detergents and Emulsifiers Annual", MC Publ. Corp., Ridgewood N. J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler "Chemische Technolgie", Band 7, C. Hauser Verlag München, 4. Aufl. 1986.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N. J.; H. v. Olphen "Intruduction to Clay Colloid Chemistry", 2nd Ed., J. Wiley & Sons, N.Y., Marsden "Solvents Guide, 2nd Ed., Interscience, N.Y. 1963; McCutcheon's "Detergents and Emulsifiers Annual", MC Publ. Corp., Ridgewood N. J.; Sisley and Wood, "Encyclopedia of Surface Active Agents", Chem. Publ. Co. Inc., N.Y. 1964; Schönfeldt "Grenzflächenaktive Äthylenoxidaddukte", Wiss. Verlagsgesell., Stuttgart 1976; Winnacker-Küchler "Chemische Technolgie", B and 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z. B. in Form einer Fertigformulierung oder als Tankmix.

Spritzpulver sind in Wasser gleichmäßig dispergierbare Präparate, die neben den Wirkstoffen außer einem Verdünnungs- oder Inertstoff noch Tenside ionischer und/oder nichtionischer Art (Netzmittel, Dispergiermittel), z. B. polyoxethylierte Alkylphenole, polyoxethylierte Fettalkohole und Fettamine, Fettalkoholpolyglykolethersulfate, Alkansulfonate oder Alkylarylsulfonate, ligninsulfonsaures Natrium, 2,2,-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes oder der Wirkstoffe in einem organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte (z. B. Blockpolymere), Alkylpolyether, Sorbitanfettsäureester, Polyoxyethylensorbitanfettsäureester oder andere Polyoxethylensorbitanester.

Stäubemittel erhält man durch Vermahlen des Wirkstoffes oder der Wirkstoffe mit fein verteilten festen Stoffen, z. B. Talkum, natürlichen Tonen, wie Kaolin, Bentonit und Pyrophyllit, oder Diatomeenerde.

Granulate können entweder durch Verdüsen des Wirkstoffes oder der Wirkstoffe auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen, auf die Oberfläche von Trägerstoffen wie Sand, Kaolinite oder von granuliertem Inertmaterial. Wasserdispergierbare Granulate werden in der Regel nach den üblichen Verfahren wie Sprühtrocknung, Wirbelbettgranulierung, Tellergranulierung, Mischung mit Hochgeschwindigkeitsmischern und Extrusion ohne festes Inertmaterial hergestellt. Auch können geeignete Wirkstoffe in der für die Herstellung von Düngemittelgranulaten üblichen Weise - gewünschtenfalls in Mischung mit Düngemitteln - granuliert werden.

Die agrochemischen Zubereitungen enthalten in der Regel 0,1 bis 99 Gew.-%, insbesondere 0,1 bis 95 Gew.-%, Wirkstoffe der Formel (I) (Antidot) oder des Herbizid/Antidot-Wirkstoffgemischs und 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-% eines Tensides.

In Spritzpulvern beträgt die Wirkstoffkonzentration z. B. etwa 10 bis 90 Gew.%, der Rest zu 100 Gew.-% besteht aus üblichen Formulierungsbestandteilen. Bei emulgierbaren Konzentraten beträgt die Wirkstoffkonzentration etwa 1 bis 80 Gew.-%. Staubförmige Formulierungen enthalten etwa 1 bis 20 Gew.-% an Wirkstoffen, versprühbare Lösungen etwa 0,2 bis 20 Gew.-% Wirkstoffe. Bei Granulaten wie wasserdispergierbaren Granulaten hängt der Wirkstoffgehalt zum Teil davon ab, ob die wirksame Verbindung flüssig oder fest vorliegt. In der Regel liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Formulierungen gegebenenfalls in üblicher Weise verdünnt, z. B. bei Spritzpulvern, emulgierbaren Konzentraten, Dispersionen und wasserdispergierbaren Granulaten mittels Wasser. Staubförmige Zubereitungen, Granulate sowie versprühbare Lösungen werden vor der Anwendung üblicherweise nicht mehr mit weiteren inerten Stoffen verdünnt. Besonders gute Wirksamkeiten der erfindungsgemäßen Mittel können erzielt werden, wenn zusätzlich zu den in den Formulierungen enthaltenen Tensiden weitere Netzmittel in Konzentrationen von 0,1 bis 0,5 Gew.-% im Tank-mix-Verfahren zugesetzt werden, z. B. nichtionische Netzmittel oder Netzmittel vom Typ der Fettalkoholpolyolethersulfate (siehe z. B. deutsche Patentanmeldung P 40 29 304.1). Mit den äußeren Bedingungen wie Temperatur, Feuchtigkeit, der Art des verwendeten Herbizids u.a. variiert die erforderliche Aufwandmenge der Safener.

Auf der Basis dieser Formulierungen lassen sich auch Kombinationen mit anderen im Pflanzenbau wirksamen Stoffe, z. B. Pestizide, wie Insektizide, Akarizide, Fungizide und Herbizide und/oder Düngemittel und/oder Wachstumsregulatoren herstellen, z. B. in Form einer Fertigformulierung oder als Tankmix.

A. Formulierungsbeispiele

a) Ein Stäubmittel wird erhalten, indem man 10 Gew.-Teile einer Verbindung der Formel (I) oder eines Wirkstoffgemischs aus einem Herbizid und eine Verbindung der Formel (I) und 90 Gew.-Teile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel (I) oder eines Wirkstoffgemischs aus einem Herbizid und einem Safener

der Formel (I), 64 Gewichtsteile kaolinhaltigen Quarz als Inertstoff, 10 Gewichtsteile ligninsulfonsaures Kaum und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel (I) oder eines Wirkstoffgemischs aus einem Herbizid und einem Safener der Formel (I), 6 Gew.-Teilen Alkylphenolpolyglykolether ($^R$Triton X 207), 3 Gew.-Teilen Isotridecanolpolyglykolether (8 EO) und 71 Gew.- Teilen paraffinischem Mineralöl (Siedebereich z. B. ca. 255 bis über 277 °C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I) oder eines Wirkstoffgemischs aus einem Herbizid und einem Safener der Formel (I), 75 Gew.-Teilen Cyclohexanon als Lösemittel und 10 Gew.-Teilen oxethyliertem Nonylphenol als Emulgator.

e) Ein in Wasser dispergierbares Granulat wird erhalten, indem man

75 Gew.-Teile einer Verbindung der Formel (I) oder eines Wirkstoffgemischs aus einem Herbizid und einem Safener der Formel (I),

10 Gew.-Teile ligninsulfonsaures Calcium,

5 Gew.-Teile Natriumlaurylsulfat,

3 Gew.-Teile Polyvinylalkohol und

7 Gew.-Teile Kaolin

mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man

25 Gew.-Teile einer Verbindung der Formel (I) oder eines Wirkstoffgemischs aus einem Herbizid und einem Safener der Formel (I),

5 Gew.-Teile 2,2'-dinaphthylmethan-6,6'-disulfonsaures Natrium,

2 Gew.-Teile oleoylmethyltaurinsaures Natrium,

1 Gew.-Teile Polyvinylalkohol,

17 Gew.-Teile Calciumcarbonat und

50 Gew.-Teile Wasser

auf einer Kolloidmühle homogensiert und vorzerkleinert, anschließend auf einer Perlmühle mahlt und die so erhaltene Suspension in einem Sprühturm mittels einer Einstoffdüse zerstäubt und trocknet.

B. Herstellungsbeispiele

a) 4-Benzthiazolyloxy-essigsäureethylester

Beispiel 2-3 aus Tabelle 2

4 g (28 mmol) 4-Hydroxybenzthiazol wurden in 210 ml absolutem Tetrahydrofuran gelöst. Bei 0 °C wurden 1,2 g (40 mmol) Natriumhydrid (80 % in Mineralöl) portionsweise zugegeben. Es wurde 0.5 Stunden bei Raumtemperatur gerührt und erneut auf 0 °C gekühlt. Bei dieser Temperatur wurde eine Lösung von 7,5 g (45 mmol) Bromessigsäureethylester zugetropft. Nach beendetem Zutropfen wurde 2 Stunden bei Raumtemperatur gerührt. Zur Aufarbeitung wurde mit 50 ml 2n Salzsäure sowie 20 ml Wasser versetzt und die Phasen getrennt. Die wäßrige Phase wurde mit Ether extrahiert und die vereinigten organischen Phasen über Magnesiumsulfat getrocknet. Nach dem Abdestillieren des Lösungsmittels wurde der Rückstand säulenchromatographisch gereinigt. Man erhielt 6 g (25 mmol, 90 % d. Th.) 4-Benzthiazolyloxy-essigsäureethylester als farbloses Öl; $^1$H-NMR ($CDCl_3$): $\delta$ [ppm] = 8,85 (s, 1H), 6,8-7,5 (m, 3H), 4,95 (s, 3H), 4,0-4,4 (q, 2H), 1,1-1,4 (t, 3H).

b) 4-Benzthiazolyloxy-essigsäure-(1-methyl)-hexylester

Beispiel 2-4 aus Tabelle 2

4,5 g (19 mmol) 4-Benzthiazolyloxy-essigsäureethylester wurden in 100 ml 2-Heptanol gelöst, mit 1 ml Titantetraisopropoxid versetzt und über Nacht bei 100 °C gerührt. Anschließend wurde das überschüssige 2-Heptanol im Ölpumpenvakuum abdestilliert und der Rückstand säulenchromatographisch gereinigt. Man erhält 2,7 g (8,8 mmol, 46 % d. Th.) 4-Benzthiazolyloxy-essigsäure-(1-methyl)-hexylester als Öl; $^1$H-NMR ($CDCl_3$): $\delta$ [ppm] = 8,82 (s, 1H), 6,75-7,6 (m, 3H), 4,8-5,2 (m, 1H), 4,9 (s, 3H), 0,6-1,8 (m, 14H).

c) 4-Benzthiazolyloxy-malonsäurediethylester

Beispiel 2-6 aus Tabelle 2

1,6 g (11 mmol) 4-Hydroxybenzthiazol wurden in 100 ml Aceton gelöst. Nach Zugabe von 7,6 g (55 mmol) Kaliumcarbonat und 6,9 g (55 mmol) Chlormalonsäurediethylester wurde 6 h auf Rückfluß erhitzt. Anschließend wurde filtriert und da Filtrat eingeengt. Das erhaltene Rohprodukt wurde chromatographisch gereinigt. Man erhielt 1,5 g (5 mmol, 45 % d. Th.) 4-Benzthiazolyloxy-malonsäurediethylester als Öl; $^1$H-

NMR ($CDCl_3$): δ [ppm] = 8,8 (s, 1H), 7,0-7,7 (m, 3H), 6,0 (s, 1H), 4,4-4,5 (q, 4H), 1,1-1,4 (t, 6H).

Die in den nachfolgenden Tabellen 1 bis 10 aufgeführten Beispiele werden in analoger Weise zu den vorstehenden Beispielen a), b) und c) sowie den weiter oben beschriebenen Verfahren erhalten. Die analogen Carbonsäuren können aus den entsprechenden Estern nach im Prinzip bekannten Methoden hergestellt werden.

Abkürzungen in Tabellen 1 bis 10: n-Alkyl oder Alkyl (n) = geradkettiges Alkyl; i-, s-, t-Alkyl = iso-, sekundär-, tertiär-Alkyl; Smp. = Schmelzpunkt

Tabelle 1: Verbindungen der Formel (1)

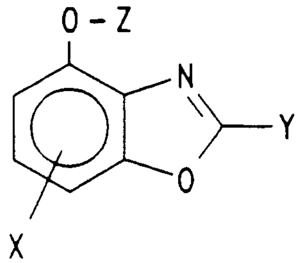

(1)

| Bsp. | X | Y | Z | Smp.[°C]; CAS <Reg.-No.> |
|---|---|---|---|---|
| 1-1 | H | H | $CH_2CO_2H$ | |
| 1-2 | H | H | $CH_2CO_2CH_3$ | |
| 1-3 | H | H | $CH_2CO_2C_2H_5$ | |
| 1-4 | H | H | $CH_2CO_2$-$CH_2$-CH = $CH_2$ | |
| 1-5 | H | H | $CH_2CO_2$-CH($CH_3$)-($CH_2$)$_4$-$CH_3$ | |
| 1-6 | H | H | $CH_2CO_2$-n-$C_8H_{17}$ (n) | |
| 1-7 | H | H | $CH_2CO_2$-$C_2H_5$-O-$C_4H_9$ (n) | |
| 1-8 | H | H | $CH_2CO_2$-CH($CH_3$)-$CH_2OCH_2CH$ = $CH_2$ | |
| 1-9 | H | H | $CH_2CO_2$-($CH_2$)$_2$-O-CO-CH($CH_3$)$_2$ | |
| 1-10 | H | H | $CH_2CO_2$-($CH_2$)$_2$-O-CO-C($CH_3$)$_3$ | |
| 1-11 | H | H | $CH_2CO_2$-($CH_2$)$_3$-O-CO-$CH_3$ | |
| 1-12 | H | H | $CH_2$-$CO_2$-($CH_2$)$_3$-O-CO-$CF_3$ | |
| 1-13 | H | H | $CH_2CO_2$ Na | |
| 1-14 | H | H | $CH_2CO_2$ K | |
| 1-15 | H | H | $CH_2CO_2NH_4$ | |
| 1-16 | H | H | $CH_2CO_2NH_2(CH_3)_2$ | |

| Bsp. | X | Y | Z | Smp.[°C]; CAS <Reg.-No.> |
|---|---|---|---|---|
| 1-17 | H | H | $CH_2$-$CO_2NH_3$(n-$C_7H_{15}$) | |
| 1-18 | H | H | $CH_2$-$CO_2NH_2(C_2H_5OH)_2$ | |
| 1-19 | H | H | $CH_2$-$CO_2NH(C_2H_5OH)_3$ | |
| 1-20 | H | H | $CH(CO_2C_2H_5)_2$ | |
| 1-21 | 7-Cl | H | $CH_2$-$CO_2H$ | |
| 1-22 | 7-Cl | H | $CH_2$-$CO_2$-$CH_3$ | |
| 1-23 | 7-Cl | H | $CH_2$-$CO_2$-$CH(CH_3)$-$(CH_2)_4$-$CH_3$ | |
| 1-24 | 7-Cl | H | $CH(CO_2C_2H_5)_2$ | |
| 1-25 | 7-Cl | $CH_3$ | $CH_2$-$CO_2H$ | |
| 1-26 | 7-Cl | $CH_3$ | $CH_2$-$CO_2$-$CH_3$ | |
| 1-27 | 7-Cl | $CH_3$ | $CH_2$-$CO_2$-$CH(CH_3)$-$(CH_2)_4$-$CH_3$ | |
| 1-28 | 7-Cl | $CH_3$ | $CH(CO_2C_2H_5)_2$ | |
| 1-29 | 7-Cl | CN | $CH_2$-$CO_2H$ | |
| 1-30 | 7-Cl | CN | $CH_2$-$CO_2$-$CH_3$ | |
| 1-31 | 7-Cl | CN | $CH_2$-$CO_2$-$CH(CH_3)$-$(CH_2)_4$-$CH_3$ | |
| 1-32 | 7-Cl | CN | $CH(CO_2C_2H_5)_2$ | |
| 1-33 | 6-F | H | $CH_2$-$CO_2H$ | |
| 1-34 | 6-F | H | $CH_2$-$CO_2CH_3$ | |
| 1-35 | 6-F | H | $CH_2$-$CO_2$-$CH(CH_3)$-$(CH_2)_4$-$CH_3$ | |
| 1-36 | 6-F | H | $CH(CO_2C_2H_5)_2$ | |
| 1-37 | H | Cl | $CH_2$-$CO_2$-$CH_3$ | |
| 1-38 | H | Cl | $CH_2$-$CO_2$-$CH(CH_3)$-$(CH_2)_4$-$CH_3$ | |
| 1-39 | H | Cl | $CH(CO_2C_2H_5)_2$ | |

Tabelle 2: Verbindungen der Formel (2)

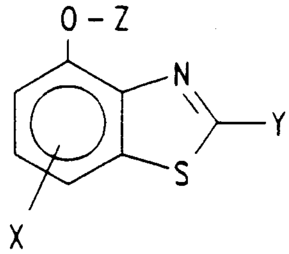

(2)

| Bsp. | X | Y | Z | Smp.[°C]; CAS <Reg.-No.> |
|---|---|---|---|---|
| 2-1 | H | H | $CH_2CO_2H$ | <30172-80-6> |
| 2-2 | H | H | $CH_2CO_2CH_3$ | |
| 2-3 | H | H | $CH_2CO_2C_2H_5$ | Öl |
| 2-4 | H | H | $CH_2$-$CO_2$-$CH(CH_3)$-$(CH_2)_4$-$CH_3$ | Öl |
| 2-5 | H | H | $CH_2$-$CO_2$-$(CH_2)_2$-O-CO-$CH(CH_3)_2$ | |
| 2-6 | H | H | $CH(CO_2C_2H_5)_2$ | |
| 2-7 | 7-Cl | H | $CH_2$-$CO_2H$ | |
| 2-8 | 7-Cl | H | $CH_2$-$CO_2$-$C_2H_5$ | 86-87 |
| 2-9 | 7-Cl | H | $CH_2$-$CO_2$-$CH(CH_3)$-$(CH_2)_4$-$CH_3$ | |
| 2-10 | 7-Cl | H | $CH(CO_2C_2H_5)_2$ | |
| 2-11 | 7-$CF_3$ | H | $CH_2$-$CO_2H$ | |
| 2-12 | 7-$CF_3$ | H | $CH_2$-$CO_2$-$C_2H_5$ | |
| 2-13 | 7-$CF_3$ | H | $CH_2$-$CO_2$-$CH(OH)_3$-$(CH_2)_4$-$CH_3$ | |
| 2-14 | 7-$CF_3$ | H | $CH(CO_2C_2H_5)_2$ | |
| 2-15 | H | Cl | $CH_2$-$CO_2H$ | |
| 2-16 | H | Cl | $CH_2$-$CO_2$-$C_2H_5$ | |
| 2-17 | H | Cl | $CH_2$-$CO_2$-$CH(CH_3)$-$(CH_2)_4$-$CH_3$ | |
| 2-18 | H | Cl | $CH(CO_2$-$C_2H_5)_2$ | |
| 2-19 | H | O-$CH_3$ | $CH_2$-$CO_2H$ | |

| Bsp. | X | Y | Z | Smp.[°C]; CAS<Reg.-No.> |
|---|---|---|---|---|
| 2-20 | H | O-$CH_3$ | CH-$CO_2$-$C_2H_5$ | |
| 2-21 | 7-Cl | O-$CH_3$ | $CH_2$-$CO_2$-CH($CH_3$)-($CH_2$)$_4$-$CH_3$ | |
| 2-22 | 7-Cl | O-$CH_3$ | CH($CO_2$-$C_2H_5$)$_2$ | |
| 2-23 | 6-Cl | F | $CH_2$-$CO_2$H | |
| 2-24 | 6-Cl | F | $CH_2$-$CO_2$-$C_2H_5$ | |
| 2-25 | 6-Cl | F | $CH_2$-$CO_2$-CH($CH_3$)-($CH_2$)$_4$-$CH_3$ | |
| 2-26 | 6-Cl | F | CH($CO_2C_2H_5$)$_2$ | |
| 2-27 | H | H | $CH_2$-$CO_2$-$CH_2$-CH = $CH_2$ | |
| 2-28 | H | H | $CH_2$-$CO_2$-n-$C_4H_9$ | |
| 2-29 | H | H | $CH_2CO_2$CH($CH_3$)-$CH_2$-<br>O$CH_2$CH = $CH_2$ | |
| 2-30 | H | H | $CH_2$-$CO_2$-($CH_2$)$_2$-O-CO-$CF_3$ | |
| 2-31 | H | H | C($CH_3$)$_2$-$CO_2$-$C_2H_5$ | |
| 2-32 | H | H | $CH_2$-$CO_2$-($CH_2$)$_2$-NH-CO-$CH_3$ | |
| 2-33 | H | H | $CH_2$-CN | |
| 2-34 | H | H | $CH_2$-CO-N($CH_3$)$_2$ | |
| 2-35 | H | H | $CH_2$-$CO_2$ K | |
| 2-36 | H | H | $CH_2$-$CO_2$ $NH_4$ | |
| 2-37 | H | H | $CH_2$-$CO_2$ $NH_2$($CH_3$)$_2$ | |
| 2-38 | H | H | $CH_2$-$CO_2$ NH($C_2H_5$OH)$_3$ | |
| 2-39 | H | H | CH[$CO_2$-CH($CH_3$)-($CH_2$)$_4$-$CH_3$]$_2$ | |
| 2-40 | H | H | CH(CN)$_2$ | |
| 2-41 | H | H | CH($CO_2$H)$_2$ | |
| 2-42 | H | H | CH(CO-$CH_3$)$_2$ | |
| 2-43 | H | H | CH($CO_2CH_3$)$_2$ | |

Tabelle 3: Verbindungen der Formel (3)

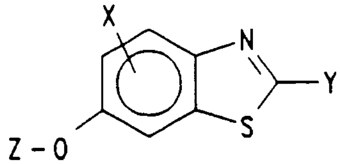

(3)

| Bsp. | X | Y | Z | Smp.[°C]; CAS <Reg.-No.> |
|---|---|---|---|---|
| 3-1 | H | H | $CH_2$-$CO_2H$ | |
| 3-2 | H | H | $CH_2$-$CO_2$-$CH_3$ | |
| 3-3 | H | H | $CH_2$-$CO_2$-$CH_2$-CH = $CH_2$ | |
| 3-4 | H | H | $CH_2$-$CO_2$-CH($CH_3$)-($CH_2$)$_4$-$CH_3$ | |
| 3-5 | H | H | $CH_2$-$CO_2$-$C_8H_{17}$ | |
| 3-6 | H | H | $CH_2$-$CO_2$-$C_2H_5$-O-$C_4H_8$ (n) | |
| 3-7 | H | H | $CH_2$-$CO_2$-($CH_2$)$_2$-O-CO-$CH_3$ | |
| 3-8 | H | H | CH($CO_2C_2H_5$)$_2$ | |
| 3-9 | H | Cl | $CH_2$-$CO_2H$ | |
| 3-10 | H | Cl | $CH_2$-$CO_2$-$C_2H_5$ | |
| 3-11 | H | Cl | $CH_2$-$CO_2$-CH($CH_3$)-($CH_2$)$_4$-$CH_3$ | |
| 3-12 | H | Cl | CH($CO_2C_2H_5$)$_2$ | |
| 3-13 | H | O-$CH_3$ | $CH_2$-$CO_2H$ | |
| 3-14 | H | O-$CH_3$ | $CH_2$-$CO_2$-$C_2H_5$ | |
| 3-15 | H | O-$CH_3$ | $CH_2$-$CO_2$-CH($CH_3$)-($CH_2$)$_4$-$CH_3$ | |
| 3-16 | H | O-$CH_3$ | CH($CO_2$-$C_2H_5$)$_2$ | |
| 3-17 | H | -CN | $CH_2$-$CO_2H$ | |
| 3-18 | H | -CN | $CH_2$-$CO_2$-$C_2H_5$ | |
| 3-19 | H | -CN | $CH_2$-$CO_2$-CH($CH_3$)-($CH_2$)$_4$-$CH_3$ | |
| 3-20 | H | -CN | CH($CO_2C_2H_5$)$_2$ | |

Tabelle 4: Verbindungen der Formel (4)

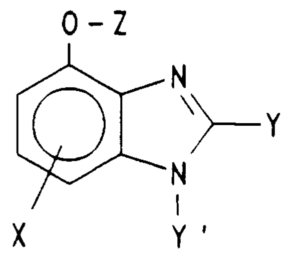

( 4 )

| Bsp. | X | Y | Y' | Z | Smp.[°C]; CAS<Reg.-No.> |
|---|---|---|---|---|---|
| 4-1 | H | H | $CH_3$ | $CH_2$-$CO_2H$ | |
| 4-2 | H | H | $CH_3$ | $CH_2$-$CO_2$-$CH_3$ | |
| 4-3 | H | H | $CH_3$ | $CH_2$-$CO_2$-$C_2H_5$ | |
| 4-4 | H | H | $CH_3$ | $CH_2$-$CO_2$-$CH_2$-CH=$CH_2$ | |
| 4-5 | H | H | $CH_3$ | $CH_2$-$CO_2$-$CH(CH_3)$-$(CH_2)_4$-$CH_3$ | |
| 4-6 | H | H | $CH_3$ | $CH_2$-$CO_2$-$C_8H_{17}$ (n) | |
| 4-7 | H | H | $CH_3$ | $CH(CO_2C_2H_5)_2$ | |
| 4-8 | H | O-$CH_3$ | $CH_3$ | $CH_2$-$CO_2H$ | |
| 4-9 | H | O-$CH_3$ | $CH_3$ | $CH_2$-$CO_2$-$C_2H_5$ | |
| 4-10 | H | O-$CH_3$ | $CH_3$ | $CH_2$-$CO_2$-$CH(CH_3)$-$(CH_2)_4$-$CH_3$ | |
| 4-11 | H | O-$CH_3$ | $CH_3$ | $CH(CO_2C_2H_5)_2$ | |
| 4-12 | H | H | $CH_3$ | $CH_2$-$CO_2H$ | |
| 4-13 | 7-Cl | H | $CH_3$ | $CH_2$-$CO_2$-$C_2H_5$ | |
| 4-14 | 7-Cl | H | $CH_3$ | $CH_2$-$CO_2CH(CH_3)$-$(CH_2)_4$-$CH_3$ | |
| 4-15 | 7-Cl | H | $CH_3$ | $CH(CO_2C_2H_5)_2$ | |
| 4-16 | H | H | -$SO_2$-$CH_3$ | $CH_2$-$CO_2H$ | |
| 4-17 | H | H | -$SO_2$-$CH_3$ | $CH_2$-$CO_2$-$C_2H_5$ | |

| Bsp. | X | Y | Y' | Z | Smp.[°C]; CAS<Reg.-No.> |
|---|---|---|---|---|---|
| 4-18 | H | H | $-SO_2-CH_3$ | $CH_2-CO_2-CH(CH_3)-(CH_2)_4-CH_3$ | |
| 4-19 | H | H | $-SO_2-CH_3$ | $CH(CO_2C_2H_5)_2$ | |
| 4-20 | H | Cl | $-SO_2-CH_3$ | $CH_2-CO_2H$ | |
| 4-21 | H | Cl | $-SO_2-CH_3$ | $CH_2-CO_2-C_2H_5$ | |
| 4-22 | H | Cl | $-SO_2-CH_3$ | $CH_2-CO_2-CH(CH_3)-(CH_2)_4-CH_3$ | |
| 4-23 | H | Cl | $-SO_2-CH_3$ | $CH(CO_2C_2H_5)_2$ | |
| 4-24 | H | H | $-CH_2-CN$ | $CH_2-CO_2H$ | |
| 4-25 | H | H | $-CH_2-CN$ | $CH_2-CO_2-C_2H_5$ | |
| 4-26 | H | H | $-CH_2-CN$ | $CH_2-CO_2-CH(CH_2)_4-CH_3$ | |
| 4-27 | H | H | $-CH_2-CN$ | $CH(CO_2C_2H_5)_2$ | |
| 4-28 | H | $CH_3$ | $-CH_2-CN$ | $CH_2-CO_2H$ | |
| 4-29 | H | $CH_3$ | $-CH_2-CN$ | $CH_2-CO_2-C_2H_5$ | |
| 4-30 | H | $CH_3$ | $-CH_2-CN$ | $CH_2-CO_2-CH(CH_3)-(CH_2)_4-CH_3$ | |
| 4-31 | H | $CH_3$ | $-CH_2-CN$ | $CH(CO_2C_2H_5)_2$ | |

Tabelle 5: Verbindungen der Formel (5)

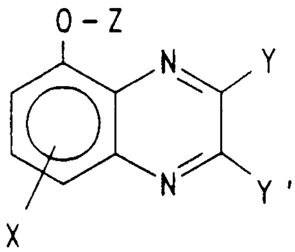

(5)

| Bsp. | X | Y | Y' | Z | Smp.[°C]; CAS<Reg.-No.> |
|---|---|---|---|---|---|
| 5-1 | H | H | H | $CH_2$-$CO_2H$ | |
| 5-2 | H | H | H | $CH_2$-$CO_2$-$CH_3$ | |
| 5-3 | H | H | H | $CH_2$-$CO_2$-$C_2H_5$ | |
| 5-4 | H | H | H | $CH_2$-$CO_2$-$CH_2$-CH=$CH_2$ | |
| 5-5 | H | H | H | $CH_2$-$CO_2$-$CH(CH_3)$-$(CH_2)_4$-$CH_3$ | |
| 5-6 | H | H | H | $CH_2$-$CO_2$-n-$C_8H_{17}$ | |
| 5-7 | H | H | H | $CH(CO_2C_2H_5)_2$ | |
| 5-8 | 8-Cl | H | H | $CH_2$-$CO_2H$ | |
| 5-9 | 8-Cl | H | H | $CH_2$-$CO_2$-$CH_3$ | |
| 5-10 | 8-Cl | H | H | $CH_2$-$CO_2$-$CH_2$-CH=$CH_2$ | |
| 5-11 | 8-Cl | H | H | $CH_2$-$CO_2$-$CH(CH_3)$-$(CH_2)_4$-$CH_3$ | |
| 5-12 | 8-Cl | H | H | $CH(COC_2H_5)_2$ | |
| 5-13 | H | $CH_3$ | H | $CH_2$-$CO_2H$ | |
| 5-14 | H | $CH_3$ | H | $CH_2$-$CO_2$-$CH_3$ | |
| 5-15 | H | $CH_3$ | H | $CH_2$-$CO_2$-$CH(CH_3)$-$(CH_2)_4$-$CH_3$ | |
| 5-16 | H | $CH_3$ | H | $CH(CO_2C_2H_5)_2$ | |
| 5-17 | H | $CH_3$ | $CH_3$ | $CH_2$-$CO_2H$ | |
| 5-18 | H | $CH_3$ | $CH_3$ | $CH_2$-$CO_2$-$CH_3$ | |
| 5-19 | H | $CH_3$ | $CH_3$ | $CH_2$-$CO_2$-$CH(CH_3)$-$(CH_2)_4$-$CH_3$ | |
| 5-20 | H | $CH_3$ | $CH_3$ | $CH(CO_2C_2H_5)_2$ | |

Tabelle 6: Verbindungen der Formel (6)

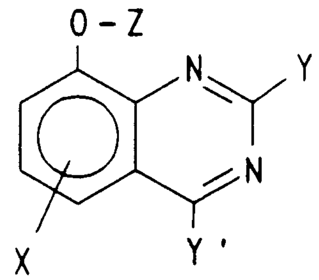

(6)

| Bsp. | X | Y | Y' | Z | Smp.[°C]; CAS<Reg.-No.> |
|---|---|---|---|---|---|
| 6-1 | H | H | H | $CH_2$-$CO_2H$ | |
| 6-2 | H | H | H | $CH_2$-$CO_2$-$CH_3$ | |
| 6-3 | H | H | H | $CH_2$-$CO_2$-$CH(CH_3)$-$(CH_2)_4$-$CH_3$ | |
| 6-4 | H | H | H | $CH(CO_2$-$C_2H_5)_2$ | |
| 6-5 | 5-Cl | H | H | $CH_2$-$CO_2H$ | |
| 6-6 | 5-Cl | H | H | $CH_2$-$CO_2$-$C_2H_5$ | |
| 6-7 | 5-Cl | H | H | $CH_2$-$CO_2$-$CH(CH_3)$-$(CH_2)_4$-$CH_3$ | |
| 6-8 | 5-Cl | H | H | $CH(CO_2$-$C_2H_5)_2$ | |
| 6-9 | 5-F | H | H | $CH_2$-$CO_2H$ | |
| 6-10 | 5-F | H | H | $CH_2$-$CO_2$-$C_2H_5$ | |
| 6-11 | 5-F | H | H | $CH_2$-$CO_2$-$CH(CH_3)$-$(CH_2)_4$-$CH_3$ | |
| 6-12 | 5-F | H | H | $CH(CO_2C_2H_5)_2$ | |
| 6-13 | 5-$CF_3$ | H | H | $CH_2$-$CO_2H$ | |
| 6-14 | 5-$CF_3$ | H | H | $CH_2$-$CO_2$-$C_2H_5$ | |
| 6-15 | 5-$CF_3$ | H | H | $CH_2$-$CO_2$-$CH(CH_3)$-$(CH_2)_4$-$CH_3$ | |
| 6-16 | 5-$CF_3$ | H | H | $CH(CO_2$-$C_2H_5)_2$ | |

Tabelle 7: Verbindungen der Formel (7)

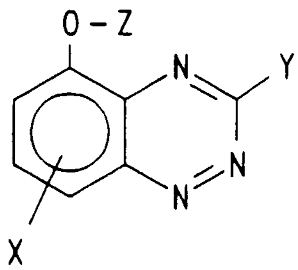

(7)

| Bsp. | X | Y | Z | Smp.[°C]; CAS <Reg.-No.> |
|---|---|---|---|---|
| 7-1 | H | H | $CH_2$-$CO_2H$ | |
| 7-2 | H | H | $CH_2$-$CO_2$-$C_2H_5$ | |
| 7-3 | H | H | $CH_2$-$CO_2$-$CH_2$-CH=$CH_2$ | |
| 7-4 | H | H | $CH_2$-$CO_2$-$CH(CH_3)$-$(CH_2)_4$-$CH_3$ | |
| 7-5 | H | H | $CH(CO_2$-$C_2H_5)_2$ | |
| 7-6 | 8-Cl | H | $CH_2$-$CO_2H$ | |
| 7-7 | 8-Cl | H | $CH_2$-$CO_2$-$C_2H_5$ | |
| 7-8 | 8-Cl | H | $CH_2$-$CO_2$-$CH(CH_3)$-$(CH_2)_4$-$CH_3$ | |
| 7-9 | 8-Cl | H | $CH(CO_2$-$C_2H_5)_2$ | |
| 7-10 | 8-F | H | $CH_2$-$CO_2H$ | |
| 7-11 | 8-F | H | $CH_2$-$CO_2$-$C_2H_5$ | |
| 7-12 | 8-F | H | $CH_2$-$CO_2$-$CH(CH_3)$-$(CH_2)_4$-$CH_3$ | |
| 7-13 | 8-F | H | $CH(CO_2$-$C_2H_5)_2$ | |
| 7-14 | 7-Cl | H | $CH_2$-$CO_2H$ | |
| 7-15 | 7-Cl | H | $CH_2$-$CO_2$-$C_2H_5$ | |
| 7-16 | 7-Cl | H | $CH_2$-$CO_2$-$CH(CH_3)$-$(CH_2)_4$-$CH_3$ | |
| 7-17 | 7-Cl | H | $CH(CO_2$-$C_2H_5)_2$ | |

Tabelle 8: Verbindungen der Formel (8)

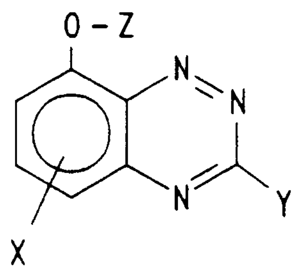

(8)

| Bsp. | X | Y | Z | Smp.[°C]; CAS<Reg.-No.> |
|---|---|---|---|---|
| 8-1 | H | H | $CH_2-CO_2H$ | |
| 8-2 | H | H | $CH_2-CO_2-C_2H_5$ | |
| 8-3 | H | H | $CH_2-CO_2-CH_2-CH=CH_2$ | |
| 8-4 | H | H | $CH_2-CO_2-CH(CH_3)-(CH_2)_4-CH_3$ | |
| 8-5 | H | H | $CH(CO_2-C_2H_5)_2$ | |
| 8-6 | 5-Cl | H | $CH_2-CO_2H$ | |
| 8-7 | 5-Cl | H | $CH_2-CO_2-C_2H_5$ | |
| 8-8 | 5-Cl | H | $CH_2-CO_2-CH(CH_3)-(CH_2)_4-CH_3$ | |
| 8-9 | 5-Cl | H | $CH(CO_2-C_2H_5)_2$ | |
| 8-10 | 5-F | H | $CH_2-CO_2H$ | |
| 8-11 | 5-F | H | $CH_2-CO_2-C_2H_5$ | |
| 8-12 | 5-F | H | $CH_2-CO_2-CH(CH_3)-(CH_2)_4-CH_3$ | |
| 8-13 | 5-F | H | $CH(CO_2-C_2H_5)_2$ | |
| 8-14 | 6-Cl | H | $CH_2-CO_2H$ | |
| 8-15 | 6-Cl | H | $CH_2-CO_2-C_2H_5$ | |
| 8-16 | 6-Cl | H | $CH_2-CO_2-CH(CH_3)-(CH_2)_4-CH_3$ | |
| 8-17 | 6-Cl | H | $CH(CO_2-C_2H_5)_2$ | |

Tabelle 9 Verbindungen der Formel (9)

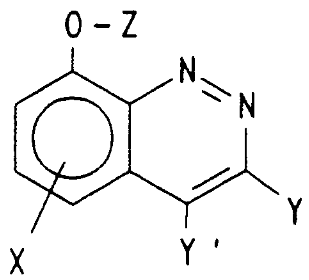

(9)

| Bsp. | X | Y | Y' | Z | Smp.[°C]; CAS <Reg.-No.> |
|---|---|---|---|---|---|
| 9-1 | 5-$CH_3$ | H | H | $CH_2$-$CO_2H$ | |
| 9-2 | 5-$CH_3$ | H | H | $CH_2$-$CO_2$-$C_2H_5$ | |
| 9-3 | 5-$CH_3$ | H | H | $CH_2$-$CO_2$-$CH(CH_3)$-$(CH_2)_4$-$CH_3$ | |
| 9-4 | 5-$CH_3$ | H | H | $CH(CO_2$-$C_2H_5)_2$ | |
| 9-5 | H | H | H | $CH_2$-$CO_2H$ | |
| 9-6 | H | H | H | $CH_2$-$CO_2$-$C_2H_5$ | |
| 9-7 | H | H | H | $CH_2$-$CO_2$-$CH(CH_3)$-$(CH_2)_4$-$CH_3$ | |
| 9-8 | H | H | H | $CH(CO_2$-$C_2H_5)_2$ | |
| 9-9 | 5-Cl | H | H | $CH_2$-$CO_2H$ | |
| 9-10 | 5-Cl | H | H | $CH_2$-$CO_2$-$C_2H_5$ | |
| 9-11 | 5-Cl | H | H | $CH_2$-$CO_2$-$CH(CH_3)$-$(CH_2)_4$-$CH_3$ | |
| 9-12 | 5-Cl | H | H | $CH(CO_2C_2H_5)_2$ | |
| 9-13 | 5-F | H | H | $CH_2$-$CO_2H$ | |
| 9-14 | 5-F | H | H | $CH_2$-$CO_2$-$C_2H_5$ | |
| 9-15 | 5-F | H | H | $CH_2$-$CO_2$-$CH(CH_3)$-$(CH_2)_4$-$CH_3$ | |
| 9-16 | 5-F | H | H | $CH(CO_2$-$C_2H_5)_2$ | |

C. Biologische Beispiele

Beispiel 1

Weizen und Gerste wurden im Gewächshaus in Plastiktöpfen bis zum 3 bis 4 Blattstadium herangezogen und dann nacheinander mit den erfindungsgemäßen Verbindungen und den getesteten Herbiziden im Nachauflaufverfahren behandelt. Die Herbizide und die Verbindungen der Formel (I) wurden dabei in Form wässriger Suspensionen bzw. Emulsionen mit einer Wasseraufwandmenge von umgerechnet 300 l/ha ausgebracht. 3 bis 4 Wochen nach der Behandlung wurden die Pflanzen visuell auf jede Art von Schädigung durch die ausgebrachten Herbizide bonitiert, wobei das Ausmaß der anhaltenden Wachstumshemmung berücksichtigt wurde. Die Bewertung erfolgte in Prozentwerten im Vergleich zu unbehandelten Kontrollen.

Selbst bei starken Überdosierungen des Herbizids werden bei den Kulturpflanzen auftretende schwere Schädigungen deutlich reduziert, geringere Schäden völlig aufgehoben. Mischungen aus Herbiziden und erfindungsgemäßen Verbindungen eignen sich deshalb in ausgezeichneter Weise zur selektiven Unkrautbekämpfung in Getreidekulturen (vgl. auch Tabelle 11).

Beispiel 2

Maispflanzen, Unkräuter und Ungräser wurden im Freiland oder im Gewächshaus in Plastiktöpfen bis zum 3 bis 4 Blattstadium herangezogen und nacheinander mit Herbiziden und erfindungsgemäßen Verbindungen der Formel (I) im Nachauflaufverfahren behandelt. Die Wirkstoffe wurden dabei in Form wässriger Suspensionen bzw. Emulsionen mit einer Wasseraufwandmenge von umgerechnet 300 l/ha ausgebracht. 4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde. Die Bewertung erfolgte in Prozentwerten im Vergleich zu unbehandelten Kontrollen.

Die Ergebnisse zeigen, daß die erfindungsgemäßen eingesetzten Verbindungen der Formel (I) starke Herbizidschäden an den Maispflanzen effektiv reduzieren können. Selbst bei starken Überdosierungen der Herbizide werden bei den Kulturpflanzen auftretende schwere Schädigungen deutlich reduziert und geringere Schäden völlig aufgehoben. Mischungen aus Herbiziden und Verbindungen der Formel (I) eignen sich deshalb in ausgezeichneter Weise zur selektiven Unkrautbekämpfung in Mais (vgl. auch Tabelle 11).

Tabelle 11

| Pflanzenschützende Wirkung erfindungsgemäßer Verbindungen | | | |
|---|---|---|---|
| Wirkstoffe | Dosis (Kg a.i./ha) | herbizide Wirkung in % | |
| | | HOVU | ZEMV |
| $H_1$ | 0,2 | 80 | - |
| $H_2$ | 0,075 | - | 75 |
| $H_1$ + $S_1$ | 0,2 + 1,25 | 23 | - |
| $H_2$ + $S_2$ | 0,075 + 1,25 | - | 30 |
| $H_1$ + $S_2$ | 0,2 + 1,25 | 15 | - |
| $H_2$ + $S_2$ | 0,075 + 1,25 | - | 35 |

Abkürzungen zu Tabelle 11:

HOVU = Hordeum vulgare, ZEMV = Zea mays
a.i. = Aktivsubstanz (bezogen auf reinen Wirkstoff = active ingredient)
$H_1$ = 2-(4-(6-Chlorbenzoxazol-2-yloxy)-phenoxypropionsäure-ethylester (Fenoxaprop-ethyl)
$H_2$ = 3-(4,6-Dimethoxypyrimidin-2-yl)-1-[3-(N-methylsulfonylamino)-2-pyridylsulfonyl]-harnstoff
$S_1$ = Beispiel 2-3 aus Tabelle 2, $S_2$ = Beispiel 2-4 aus Tabelle 2

**Patentansprüche**

1. Verwendung von Verbindungen der Formel (I) oder deren Salze,

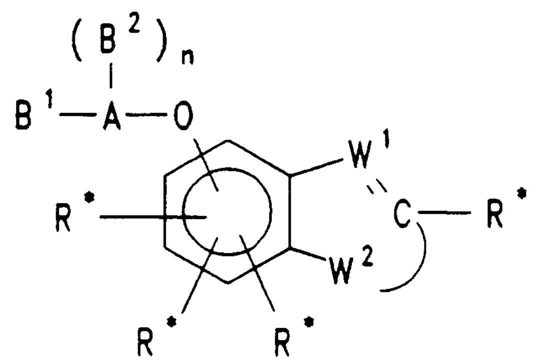

(I)

worin

W¹ N oder CR* und

W² O, S, NH, NR**, N = N, N = CR* oder CR* = N bedeuten und die Summe der Heteroringatome aus den Gruppen W¹ und W² 2 oder 3 ist, sowie

A ein divalenter Kohlenwasserstoffrest mit 1 bis 12 C-Atomen,

B¹, B² jeweils unabhängig voneinander Carboxy, Derivate der Carboxygruppe aus der Gruppe Ester, Thioester, Nitrile, Imide, Anhydride, Halogenide, Amide, Hydrazide, Imidsäure, Hydrazonsäure, Hydroxamsäure, Hydroximsäure, Amidin, Amidoxid und Derivate der letztgenannten 9 funktionellen Reste, die am N-Atom bzw. der Hydroxygruppe, weiter substituiert sind, oder Formyl, Derivate der Formylgruppe aus der Gruppe Acetale, Thioacetale, Imine, Hydrazon, Oxim und Derivate der letztgenannten 3 funktionellen Reste, die am N-Atom bzw. an der Hydroxygruppe weiter substituiert sind, oder ein Acylrest der Formel -CO-R,

worin

R ein aliphatischer, araliphatischer oder aromatischer Rest ist, der noch weitere funktionelle Gruppen enthalten und insgesamt bis zu 24 C-Atome aufweisen kann,

n 0 oder 1,

R* jeweils unabhängig von den anderen Resten R* Wasserstoff, Halogen, Halogen-($C_1$-$C_8$)-alkyl, Halogen-($C_1$-$C_8$)-alkoxy, ($C_1$-$C_8$)-Alkyl, ($C_1$-$C_8$)-Alkoxy, Nitro, Amino oder Amino, das durch ein oder zwei Reste aus der Gruppe ($C_1$-$C_4$)-alkyl und Acyl mit 1 bis 10 C-Atomen substituiert ist, oder Cyano, ($C_1$-$C_8$)-Alkylthio oder ($C_1$-$C_8$)-Alkylsulfonyl oder gegebenenfalls substituiertes Phenyl, und

R** ($C_1$-$C_8$)-Alkyl, Halogen-($C_1$-$C_8$)-alkyl, ($C_1$-$C_8$)-Alkylthio, ($C_1$-$C_8$)-Alkoxy, Halogen-($C_1$-$C_8$)-alkoxy, ($C_1$-$C_8$)-Alkylsulfonyl, Cyano-($C_1$-$C_8$)-alkyl, [($C_1$-$C_8$)-Alkyl]-carbonyl oder [($C_1$-$C_8$)-Alkoxy]-carbonyl

bedeuten, als Safener zur Vermeidung oder Reduzierung phytotoxischer Effekte von Pflanzenschutzmitteln an Kulturpflanzen.

2. Verwendung nach Anspruch 1, dadurch gekennzeichnet, daß

A ($C_1$-$C_6$)-Alkandiyl oder ($C_3$-$C_8$)-Alkendiyl,

B¹, B² jeweils unabhängig voneinander einen Rest der Formel $-COOR^1$, $-COSR^1$, $-CO-ON = CR^2R^3$, $-CO-NR^4N = CR^2R^3$, $-CONR^1R^4$, $-CO-R^1$,

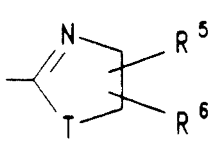, 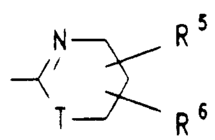, 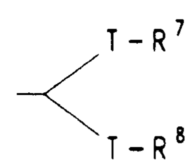 oder 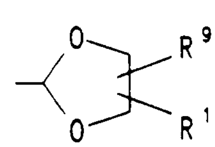,

$R^1$ Wasserstoff, $(C_1\text{-}C_{18})$-Alkyl, $(C_3\text{-}C_{12})$-Cycloalkyl, $(C_3\text{-}C_{12})$-Heterocyclyl, $(C_2\text{-}C_{18})$-Alkenyl, $(C_2\text{-}C_8)$-Alkinyl, Aryl oder Heteroaryl, wobei jeder der vorstehenden C-haltigen Reste unsubstituiert oder durch einen oder mehrere, gleiche oder verschiedene Substituenten aus der Gruppe Halogen, Halogen- $(C_1\text{-}C_8)$-alkoxy, Nitro, Cyano, Hydroxy, $(C_1\text{-}C_8)$-Alkoxy und $(C_1\text{-}C_8)$-Alkoxy, worin eine oder mehrere nicht nebeneinanderliegende $CH_2$-Gruppen durch Sauerstoff ersetzt sind, und $(C_1\text{-}C_8)$-Alkyl und Halo-$(C_1\text{-}C_8)$-alkyl, letztere beiden nur bei cyclischen Resten, $(C_1\text{-}C_8)$-Alkylthio, $(C_1\text{-}C_6)$-Alkylsulfinyl, $(C_1\text{-}C_6)$-Alkylsulfonyl, $(C_2\text{-}C_8)$-Alkenylthio, $(C_2\text{-}C_8)$-Alkinylthio, $(C_2\text{-}C_8)$-Alkenyloxy, $(C_2\text{-}C_8)$-Alkinyloxy, $(C_3\text{-}C_7)$-Cycloalkyl, $(C_3\text{-}C_7)$-Cycloalkoxy, $(C_1\text{-}C_8)$-Alkoxycarbonyl, $(C_2\text{-}C_8)$-Alkenyloxycarbonyl, $(C_2\text{-}C_8)$-Alkinyloxycarbonyl, $(C_1\text{-}C_8)$-Alkylthiocarbonyl, $(C_1\text{-}C_8)$-Alkylcarbonyl, Formyl, $(C_2\text{-}C_8)$-Alkenylcarbonyl, $(C_2\text{-}C_8)$-Alkinylcarbonyl, Hydroxyimino, $(C_1\text{-}C_4)$-Alkylimino, $(C_1\text{-}C_4)$-Alkoxyimino, $(C_1\text{-}C_8)$-Alkylcarbonylamino, $(C_2\text{-}C_8)$-Alkenylcarbonylamino, $(C_2\text{-}C_8)$-Alkinylcarbonylamino, Carbamoyl, $(C_1\text{-}C_8)$-Alkylcarbamoyl, Di-$(C_1\text{-}C_6)$-Alkylcarbamoyl, $(C_2\text{-}C_6)$-Alkenylcarbamoyl, $(C_2\text{-}C_6)$-Alkinylcarbamoyl, $(C_1\text{-}C_8)$-Alkoxycarbonylamino, $(C_1\text{-}C_8)$-Alkyl-aminocarbonylamino, Di-$(C_1\text{-}C_8)$-Alkylaminocarbonylamino, $(C_1\text{-}C_8)$-Alkoxycarbonyloxy, $(C_1\text{-}C_8)$-Alkylcarbonyloxy, das unsubstituiert oder durch Halogen, Nitro, $(C_1\text{-}C_4)$-Alkoxy oder gegebenenfalls substituiertes Phenyl substituiert ist, $(C_2\text{-}C_6)$-Alkenylcarbonyloxy, $(C_2\text{-}C_6)$-Alkinylcarbonyloxy, Phenyl, Phenyl-$(C_1\text{-}C_6)$-alkoxy, Phenyl-$(C_1\text{-}C_6)$-alkoxycarbonyl, Phenoxy, Phenoxy-$(C_1\text{-}C_6)$-alkoxy, Phenoxycarbonyl, Phenoxy-$(C_1\text{-}C_6)$-alkoxycarbonyl, Phenylcarbonyloxy, Phenylcarbonylamino, Phenyl-$(C_1\text{-}C_6)$-alkylcarbonylamino, wobei die letztgenannten 10 Reste im Phenylring unsubstituiert oder ein- oder mehrfach durch gleiche oder verschiedene Reste aus der Gruppe Halogen, $(C_1\text{-}C_4)$-Alkyl, $(C_1\text{-}C_4)$-Alkoxy, $(C_1\text{-}C_4)$-Halogenalkyl, $(C_1\text{-}C_4)$-Halogenalkoxy und Nitro substituiert sind, und Reste der Formeln $-SiR^2R^3R^4$, $-O-SiR^2R^3R^4$, $R^2R^3R^4Si-(C_1\text{-}C_6)$-alkoxy, $-CO-O-NR^2R^3$, $-O-N = CR^2R^3$, $-N = CR^2R^3$, $O-(CH_2)_m-CH-(OR^2)OR^3$, R'O-CHR''-CH(OR')-$(C_1\text{-}C_6)$-alkoxy, $-NR^2R^3$, $-CO-NR^2R^3$, $-O-CO-NR^2R^3$ und einen drei- bis siebengliedrigen, gegebenenfalls benzokondensierten und gegebenenfalls substituierten gesättigten oder ungesättigten heterocyclischen Rest mit bis zu drei gleichen oder verschiedenen Heteroatomen aus der Reihe S, O und N substituiert ist,

R' unabhängig voneinander $(C_1\text{-}C_4)$-Alkyl oder paarweise zusammen einen $(C_1\text{-}C_6)$-Alkandiylrest,

R'' Wasserstoff oder $(C_1\text{-}C_4)$-Alkyl,

$R^2$ und $R^3$ jeweils unabhängig voneinander Wasserstoff, gegebenenfalls substituiertes $(C_1\text{-}C_6)$-Alkyl, $(C_2\text{-}C_6)$-Alkenyl, $(C_2\text{-}C_6)$-Alkinyl, Phenyl-$(C_1\text{-}C_4)$-alkyl oder Phenyl, wobei die letztgenannten beiden Reste im Phenylrest unsubstituiert oder substituiert sind, oder $R^2$ und $R^3$ gemeinsam eine gegebenenfalls substituierte $(C_2\text{-}C_6)$-Alkandiylkette,

$R^4$ Wasserstoff oder gegebenenfalls substituiertes $(C_1\text{-}C_4)$-Alkyl oder

$R^1$ und $R^4$ gemeinsam eine Alkandiylkette mit 2 bis 5 C-Atomen, in der eine $CH_2$-Gruppe gegebenenfalls durch O, NH oder N-$(C_1\text{-}C_4)$-Alkyl ersetzt sein kann,

$R^5$ und $R^6$ gleich oder verschieden sind und unabhängig voneinander Wasserstoff oder $(C_1\text{-}C_6)$-Alkyl,

$R^7$ und $R^8$ gleich oder verschieden sind und unabhängig voneinander Wasserstoff oder $(C_1\text{-}C_6)$-Alkyl, das durch Halogen, $(C_1\text{-}C_4)$-Alkoxy oder Phenyl substituiert sein kann,

$R^9$ und $R^{10}$ gleich oder verschieden sind und unabhängig voneinander Wasserstoff oder $(C_1\text{-}C_6)$-Alkyl, das durch Halogen, $(C_1\text{-}C_4)$-Alkoxy oder OH substituiert sein kann,

T unabhängig voneinander Sauerstoff oder Schwefel und

m eine ganze Zahl von 0 bis 6

bedeuten.

**3.** Verwendung nach Anspruch 1 oder 2, dadurch gekennzeichnet, daß

A $CH_2$, $CH(CH_3)$, $CH_2CH_2$, $CH_2CH_2CH_2$ oder $C(CH_3)_2$,

R* jeweils unabhängig von den anderen Resten R* Wasserstoff, Halogen, Halogen-$(C_1\text{-}C_6)$-alkyl, Halogen-$(C_1\text{-}C_6)$-alkoxy, $(C_1\text{-}C_8)$-Alkyl, $(C_1\text{-}C_8)$-Alkoxy, Nitro, Amino oder Amino, das durch ein oder zwei Reste aus der Gruppe $(C_1\text{-}C_4)$-alkyl und Acyl mit 1 bis 10 C-Atomen substituiert ist, oder Cyano, $(C_1\text{-}C_6)$-Alkylthio oder $(C_1\text{-}C_8)$-Alkylsulfonyl,

$R^1$ Wasserstoff, ($C_1$-$C_{12}$)-Alkyl, ($C_3$-$C_8$)-Cycloalkyl, ($C_3$-$C_8$)-Heterocyclyl, ($C_2$-$C_{12}$)-Alkenyl, ($C_2$-$C_8$)-Alkinyl oder Phenyl, wobei jeder der vorstehenden C-haltigen Reste unsubstituiert oder durch einen oder mehrere gleiche oder verschiedene Substituenten aus der Gruppe Halogen, Halogen-($C_1$-$C_8$)-alkoxy, Nitro, Cyano, Hydroxy, ($C_1$-$C_8$)-Alkoxy und ($C_1$-$C_8$)-Alkoxy, worin eine oder mehrere nicht nebeneinanderliegende $CH_2$-Gruppen durch Sauerstoff ersetzt sind, und ($C_1$-$C_8$)-Alkyl und Halo-($C_1$-$C_8$)-alkyl, letzte beiden nur bei cyclischen Resten, ($C_1$-$C_8$)-Alkylthio, ($C_1$-$C_6$)-Alkylsulfinyl, ($C_1$-$C_6$)-Alkylsulfonyl, ($C_2$-$C_8$)-Alkenylthio, ($C_2$-$C_8$)-Alkinylthio, ($C_2$-$C_8$)-Alkenyloxy, ($C_2$-$C_8$)-Alkinyloxy, ($C_3$-$C_7$)-Cycloalkyl, ($C_3$-$C_7$)-Cycloalkoxy, ($C_1$-$C_8$)-Alkoxycarbonyl, ($C_2$-$C_8$)-Alkenyloxycarbonyl, ($C_2$-$C_8$)-Alkinyloxycarbonyl, ($C_1$-$C_8$)-Alkylthiocarbonyl, ($C_1$-$C_8$)-Alkylcarbonyl, ($C_2$-$C_8$)-Alkenylcarbonyl, ($C_2$-$C_8$)-Alkinylcarbonyl, Hydroxyimino, ($C_1$-$C_4$)-Alkylimino, ($C_1$-$C_4$)-Alkoxyimino, ($C_1$-$C_8$)-Alkylcarbonylamino, ($C_2$-$C_8$)-Alkenylcarbonylamino, ($C_2$-$C_8$)-Alkinylcarbonylamino, Carbamoyl, ($C_1$-$C_8$)-Alkylcarbamoyl, Di-($C_1$-$C_6$)-Alkylcarbamoyl, ($C_2$-$C_6$)-Alkenylcarbamoyl, ($C_2$-$C_6$)-Alkinylcarbamoyl-($C_1$-$C_8$)-Alkoxycarbonylamino, ($C_1$-$C_8$)-Alkyl-amino-carbonylamino, ($C_1$-$C_8$)-Alkoxycarbonyloxy, ($C_1$-$C_8$)-Alkylcarbonyloxy, das unsubstituiert oder durch Halogen, Nitro, ($C_1$-$C_4$)-Alkoxy oder gegebenenfalls substituiertes Phenyl substituiert ist, ($C_2$-$C_6$)-Alkenylcarbonyloxy, ($C_2$-$C_6$)-Alkinylcarbonyloxy, Phenyl, Phenyl-($C_1$-$C_6$)-alkoxy, Phenyl-($C_1$-$C_6$)-alkoxycarbonyl, Phenoxy, Phenoxy-($C_1$-$C_6$)-alkoxy, Phenoxycarbonyl, Phenoxy-($C_1$-$C_6$)-alkoxycarbonyl, Phenylcarbonyloxy, Phenylcarbonylamino, Phenyl-($C_1$-$C_6$)-alkylcarbonylamino, wobei die letztgenannten 10 Reste im Phenylring unsubstituiert oder ein- oder mehrfach durch gleiche oder verschiedene Reste aus der Gruppe Halogen, ($C_1$-$C_4$)-Alkyl, ($C_1$-$C_4$)-Alkoxy, ($C_1$-$C_4$)-Halogenalkyl, ($C_1$-$C_4$)-Halogenalkoxy und Nitro substituiert sind, und Reste der Formeln $-SiR^2R^3R^4$, $-O-SiR^2R^3R^4$, $R^2R^3R^4Si$-($C_1$-$C_6$)-alkoxy, $-CO-O-NR^2R^3$, $-O-N=CR^2R^3$, $-N=CR^2R^3$, $-NR^2R^3$, $-CONR^2R^3$, $-OCONR^2R^3$, $-O-(CH_2)_m-CH(OR^2)OR^3$, R'O-CHR''-CH(OR')-($C_1$-$C_6$)-alkoxy und einen fünf- bis sechsgliedrigen, gegebenenfalls benzokondensierten und gegebenenfalls substituierten gesättigten oder ungesättigten heterocyclischen Rest mit bis zu drei gleichen oder verschiedenen Heteroatomen aus der Reihe S, O und N substituiert ist,

R' unabhängig voneinander ($C_1$-$C_4$)-Alkyl oder paarweise zusammen einen ($C_1$-$C_6$)-Alkandiylrest,

R'' Wasserstoff oder ($C_1$-$C_4$)-Alkyl,

$R^2$ und $R^3$ unabhängig voneinander Wasserstoff, ($C_1$-$C_4$)-Alkyl oder gegebenenfalls substituiertes Phenyl oder gemeinsam eine unverzweigte ($C_2$-$C_4$)-Alkandiylkette und

m eine ganze Zahl von 0 bis 6 bedeuten.

**4.** Verwendung nach einem der Ansprüche 1 bis 3, dadurch gekennzeichnet, daß mindestens zwei Reste R* am Benzolring Wasserstoff bedeuten und

$R^1$ Wasserstoff, ($C_1$-$C_{12}$)-Alkyl, ($C_3$-$C_8$)-Cycloalkyl, ($C_2$-$C_{12}$)-Alkenyl, ($C_2$-$C_8$)-Alkinyl, wobei jeder der vorstehenden C-haltigen Reste unsubstituiert oder durch einen oder mehrere Substituenten aus der Gruppe enthaltend Hydroxy, ($C_1$-$C_8$)-Alkoxy, Alkoxypolyalkylenoxy mit insgesamt 2 bis 8 C-Atomen, ($C_1$-$C_4$)-Alkylthio, ($C_2$-$C_4$)-Alkenylthio, ($C_2$-$C_4$)-Alkinylthio, ($C_2$-$C_4$)-Alkenyloxy, ($C_2$-$C_4$)-Alkinyloxy, Mono- und Di-($C_1$-$C_2$)-alkylamino, ($C_1$-$C_4$)-Alkoxycarbonyl, ($C_2$-$C_4$)-Alkenyloxycarbonyl, ($C_2$-$C_4$)-Alkinyloxycarbonyl, ($C_1$-$C_4$)-Alkylcarbonyl, ($C_2$-$C_4$)-Alkenylcarbonyl, ($C_2$-$C_4$)-Alkinylcarbonyl, ($C_1$-$C_4$)-Alkylcarbonylamino, ($C_2$-$C_4$)-Alkenylcarbonylamino, Carbamoyl, ($C_1$-$C_8$)-Alkylcarbamoyl, Di-($C_1$-$C_6$)-Alkylcarbamoyl, ($C_1$-$C_4$)-Alkoxycarbonyloxy, ($C_1$-$C_4$)-Alkylcarbonyloxy, das unsubstituiert oder durch ein oder zwei Reste aus der Gruppe Halogen und ($C_1$-$C_4$)-Alkoxy substituiert ist, und ($C_2$-$C_4$)-Alkenylcarbonyloxy, ($C_2$-$C_4$)-Alkinylcarbonyloxy, Phenyl, Phenyl-($C_1$-$C_4$)-alkoxy, Phenyl-($C_1$-$C_4$)-alkoxycarbonyl, Phenoxy, Phenoxy-($C_1$-$C_4$)-alkoxy, Phenoxycarbonyl, Phenoxy-($C_1$-$C_4$)-alkoxycarbonyl, Phenylcarbonyloxy, wobei die letztgenannten 8 Reste im Phenylring unsubstituiert oder durch einen oder mehrere Reste aus der Gruppe enthaltend Halogen, ($C_1$-$C_2$)-Alkyl, ($C_1$-$C_2$)-Alkoxy, ($C_1$-$C_2$)-Halogenalkyl, ($C_1$-$C_2$)-Halogenalkoxy und Nitro substituiert sind, und Reste der Formeln $-SiR^2R^3R^4$, $O-SiR^2R^3R^4$, $R^2R^3R^4Si$-($C_1$-$C_4$)-alkoxy, $-O-N=CR^2R^3$, $-N=CR^2R^3$, $O-(CH_2)_m-CH(OR^2)OR^3$, R'O-CHR''-CH-(OR')-($C_1$-$C_6$)-alkoxy, $NR^2R^3$, $-CONR^2R^3$ und $-OCONR^2R^3$ substituiert ist,

R' unabhängig voneinander ($C_1$-$C_4$)-Alkyl oder paarweise zusammen einen ($C_1$-$C_6$)-

Alkandiylrest und

| | |
|---|---|
| R" | Wasserstoff oder ($C_1$-$C_4$)-Alkyl, |
| $R^2$ und $R^3$ | gleich oder verschieden sind und unabhängig voneinander Wasserstoff, ($C_1$-$C_4$)-Alkyl oder gegebenenfalls substituiertes Phenyl, oder gemeinsam eine gegebenenfalls substituierte ($C_2$-$C_4$)-Alkandiylkette, |
| $R^5$ und $R^6$ | gleich oder verschieden sind und unabhängig voneinander Wasserstoff oder ($C_1$-$C_4$)-Alkyl, |
| $R^7$ und $R^8$ | gleich oder verschieden sind und unabhängig voneinander Wasserstoff oder ($C_1$-$C_4$)-Alkyl, |
| $R^9$ und $R^{10}$ | unabhängig voneinander Wasserstoff oder ($C_1$-$C_4$)-Alkyl und |
| m | eine ganze Zahl von 0 bis 2 |

bedeuten.

5. Verbindungen der Formel (I) oder deren Salze, wie sie in einem der Ansprüche 1 bis 4 definiert sind, ausgenommen bekannte Verbindungen der Formel (I) oder bekannte Salze von Verbindungen der Formel (I).

6. Verfahren zur Herstellung von Verbindungen der Formel (I) oder deren Salze nach Anspruch 5, dadurch gekennzeichnet, daß man

a) eine Verbindung der Formel

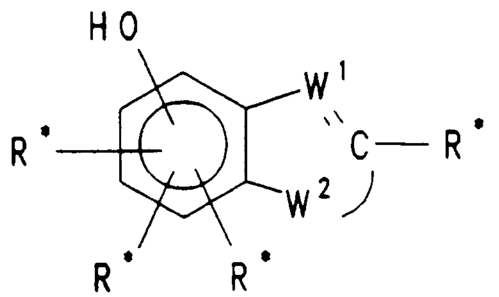

(II)

mit einer Verbindung der Formel (III),

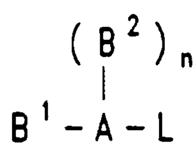

(III)

worin n eine Abgangsgruppe bedeutet, umsetzt oder

b) im Falle n = 0 und $B^1$ = Derivat der Carboxygruppe, eine Verbindung der Formel (IV)

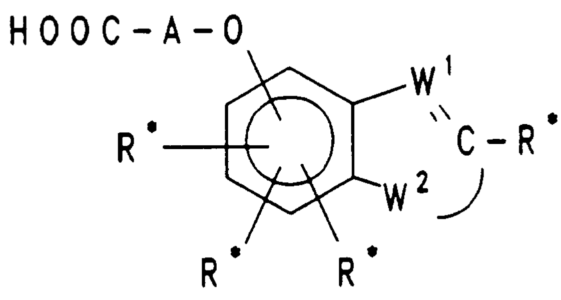

(IV)

zu Säurederivaten der Formel (I), n = 0 umsetzt oder

c) im Falle, daß $B^1$ und $B^2$ Carbonsäurederivatgruppen sind eine Verbindung der Formel (I'),

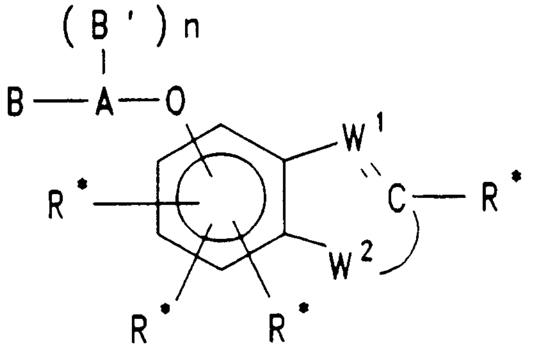

(I')

worin B und B' analog $B^1$ und $B^2$ definiert sind, zu Verbindungen der Formel (I) umestert bzw. amidiert,

wobei in den Formeln (II), (III), (IV) und (I') die Reste A, $B^1$, $B^2$, n, $R^*$, $W^1$ und $W^2$ wie in Formel (I) definiert sind.

**7.** Herbizid-Safener-Kombination, dadurch gekennzeichnet, daß sie einen Safener der Formel (I) oder dessen Salz nach einem der Ansprüche 1 bis 5 und mindestens ein Herbizid enthält.

**8.** Pflanzenbehandlungsmittel, dadurch gekennzeichnet, daß es einen Safener der Formel (I) oder dessen Salz und übliche Formulierungshilfsmittel enthält.

**9.** Verfahren zum Schutz von Kulturpflanzen vor phytotoxischen Nebenwirkungen von Herbiziden, dadurch gekennzeichnet, daß eine wirksame Menge eines Safeners der Formel (I) oder dessen Salz nach einem der Ansprüche 1 bis 5 vor, nach oder gleichzeitig mit dem Herbizid auf die Pflanzen, Pflanzenteile, Pflanzensamen oder die Anbauflächen appliziert wird.

**10.** Verfahren zur selektiven Bekämpfung von Schadpflanzen in Nutzpflanzenkulturen, dadurch gekennzeichnet, daß eine Herbizid-Safener-Kombination nach Anspruch 7 auf die Anbauflächen im Vor- und Nachauflaufverfahren appliziert wird.